# EUROPEAN PATENT APPLICATION

(11) **EP 1 586 902 A2**
(43) Date of publication of application: **19.10.2005**
(21) Application number: 05006565.5
(22) Date of filing: 24.03.2005
(51) Int. Cl.: G01N 33/566, G01N 33/74, C12Q 1/68, A61P 3/00

(54) **ANGPTL4/FIAF as marker for PPARdelta modulation**

(30) Priority: 30.03.2004 EP 04101305; 09.12.2004 EP 04106445; 27.01.2005 EP 05100513
(71) Applicant: F.HOFFMANN-LA ROCHE AG, 4070 Basel (CH)
(72) Inventor: Clerc, Roger C., 4102 Binningen (CH); Ebeling, Martin, 79639 Grenzach-Wyhlen (DE); Gardes, Christophe, 68870 Bartenheim (FR); Meyer, Markus, 79395 Neuenburg (DE); Wright, Matthew Blake, 4058 Basel (CH)

(57) **Abstract**

The present invention relates to ANGPTL4 as biomarkers for PPARdelta activity, and to methods of diagnosing a disease linked to dysregulation of PPARdelta activity such as for example dyslipidemia, obesity or insulin resistance, methods of monitoring the treatment of patients suffering from a disease linked to dysregulation of PPARdelta activity and methods of identifying compounds which modulate PPARdelta activity.

SEQUENCE LISTING

<110> F. Hoffmann-La Roche AG

<120> ANGPTL4/FIAF as Marker for PPAR modulation

<130> 22517

<160> 39

<170> PatentIn version 3.2

<210> 1
<211> 1883
<212> DNA
<213> Mus musculus

<220>
<221> Angiopoietin-like protein 4 (ANGPTL4)
<222> (1)..(1870)
<223> representative cDNA (complete cds) (BC021343 as of 25.03.2004),

for gene under UniGene ID Mm.196189

<400> 1
ccacgcgtcc ggctacgggc tccagatctt cttctgcacc agagcaagtc taagtctgag 60

ccggctcccc cagaactcca gctgctgggt cttgaactcc tgcgttccgg agtcctagcg 120

ttgctgcacc caaggccacc cccagaatca tgcgctgcgc tccgacagca ggcgctgccc 180

tggtgctatg cgcggctact gcggggcttt tgagcgcgca agggcgccct gcacagccag 240

agccaccgcg ctttgcatcc tgggacgaga tgaacttgct ggctcacggg ctgctacagc 300

tcggccatgg gctgcgcgaa cacgtggagc gcacccgtgg gcagctgggc gcgctggagc 360

gccgcatggc tgcctgtggt aacgcttgtc aggggcccaa gggaaaagat gcacccttca 420

aagactccga ggatagagtc cctgaaggcc agactcctga gactctgcag agtttgcaga 480

ctcagctcaa ggctcaaaac agcaagatcc agcaattgtt ccagaaggtg gcccagcagc 540

agagatacct atcaaagcag aatctgagaa tacagaatct tcagagccag atagacctct 600

tggcccccac gcacctagac aatggagtag acaagacttc gaggggaaag aggcttccca 660

agatgaccca gctcattggc ttgactccca acgccaccca cttacacagg ccgccccggg 720

actgccagga actcttccaa gaaggggaga ggcacagtgg acttttccag atccagcctc 780

tggggtctcc accatttttg gtcaactgtg agatgacttc agatggaggc tggacagtga 840

ttcagagacg cctgaacggc tctgtggact tcaaccagtc ctgggaagcc tacaaggatg 900

gcttcggaga tccccaaggc gagttctggc tgggcctgga aaagatgcac agcatcacag 960

ggaaccgagg aagccaattg gctgtgcagc tccaggactg ggatggcaat gccaaattgc 1020

tccaatttcc catccatttg gggggtgagg acacagccta cagcctgcag ctcactgagc 1080

ccacggccaa tgagctgggt gccaccaatg tttcccccaa tggcctttcc ctgcccttct 1140

ctacttggga ccaagaccat gacctccgtg gggaccttaa ctgtgccaag agcctctctg 1200

gtggctggtg gtttggtacc tgtagccatt ccaatctcaa tggacaatac ttccactcta 1260

tcccacggca acggcaggag cgtaaaaagg gtatcttctg gaaaacatgg aagggccgct 1320

actatcctct gcaggctacc accctgctga tccagcccat ggaggctaca gcagcctctt 1380

agcctcctca ctggagcctg gttccaggcc taagaagaca gtgactttgg ttgtggccct 1440

gagatttggc cattctctgc tgggggcagg agctctaagt agggctatct gcgtcttgtg 1500

gacaaagaag aagcccgtaa ctggagagac tggaggaccc cttttccgtg ttggggtctg 1560

caagcattgt tgtctgaaac agtcagagca acaggaaaca aatggcccag atccagaaaa 1620

catgggctcg aggggcactg aatatcactt ctcgcctacc agagaagttg gggatgcaga 1680

gggaccacta cagtccaact agctgggccc ttaatggcgg actcagtcat attgactgac 1740

tggagacagg gtgccaggag ccctggatac actcatggtg ctgttgtagg tgctgtggat 1800

gcacaggtgc taactgtggt tcccaggcac aactcacagc attcttacaa taaaaacaac 1860

ctcagaacaa aaaaaaaaaa aaa 1883

<210> 2
<211> 1858
<212> DNA
<213> Mus musculus

<220>
<221> Angiopoietin-like protein 4 (ANGPTL4)
<222> (1)..(1858)
<223> representative cDNA (NM_020581, as of 25.03.2004),

for gene under UniGene ID Mm.196189

<400> 2
gcaccagagc aagtctaagt ctgagccggc tcccccagaa ctccagctgc tgggtcttga 60

actcctgcgt tccggagtcc tagcgttgct gcacccaagg ccacccccag aatcatgcgc 120

tgcgctccga cagcaggcgc tgccctggtg ctatgcgcgg ctactgcggg gcttttgagc 180

gctcaagggc gccctgcaca gccagagcca ccgcgctttg catcctggga cgagatgaac 240

ttgctggctc acgggctgct acagctcggc catgggctgc gcgaacacgt ggagcgcacc 300

cgtgggcagc tgggcgcgct ggagcgccgc atggctgcct gtggtaacgc ttgtcagggg 360

cccaagggaa aagatgcacc cttcaaagac tccgaggata gagtccctga aggccagact 420

cctgagactc tgcagagttt gcagactcag ctcaaggctc aaaacagcaa gatccagcaa 480

ttgttccaga aggtggccca gcagcagaga tacctatcaa agcagaatct gagaatacag 540

aatcttcaga gccagataga cctcttggcc cccacgcacc tggacaatgg agtagacaag 600

acttcgaggg gaaagaagct ttccaagatg acccagctca ttggcttgac ttccaacgcc 660

acccacttac acaggccggc ccgggactgc caggaactct tccaagaagg ggagaggcac 720

agtggacttt tccagatcca gcctctgggg tctccaccat ttttggtcaa ctgtgagatg 780
acttcagatg gaggctggac agtgattcag agacgcctga acggctctgt ggacttcaac 840
cagtcctggg aagcctacaa ggatggcttc ggagatcccc aaggcgagtt ctggctgggc 900
ctggaaaaga tgcacagcat cacaggggac cgaggaagcc aattggctgt gcagctccag 960
gactgggatg gcaatgccaa attgctccaa tttcccatcc atttgggggg tgaggacaca 1020
gcctacagcc tgcagctcac tgagcccacg gccaatgagc tgggtgccac caatgtttcc 1080
cccaatggcc tttccctgcc cttctctact tgggaccaag accatgacct ccgtggggac 1140
cttaactgtg ccaagagcct ctctggtggc tggtggtttg gtacctgtag ccattccaat 1200
ctcaatggac aatacttcca ctctatccca cggcaacggc aggagcgtaa aaagggtatc 1260
ttctggaaaa catggaaggg ccgctactat cctctgcagg ctaccaccct gttgatccag 1320
cccatggagg ctacagcagc ctcttagcct cctcactgga gcctggttcc aggcctaaga 1380
agacagtgac tttggttgtg gccctgagat ttggccattc tctgctgggg gcaggagctc 1440
taagtagggc tatctgcgtc ttgtggacaa agaagaagcc cgtaactgga gagactggag 1500
gacccctttt ccgtgttggg gtctgcaagc attgttgtct gaaacagtca gagcaacagg 1560
aaacaaatgg cccagatcca gaaaacatgg gctcgagggg cactgaatat cacttctcgc 1620
ctaccagaga agttggggat gcagagggac cactacagtc caactagctg ggcccttaat 1680
ggcggactca gtcatattga ctgactggag acagggtgcc aggagccctg gatacactca 1740
tggtgctgtt gtaggtgctg tggatgcaca ggtgctaact gtggttccca ggcacagctc 1800
acagcattct tacaataaaa acaacctcag aacaaaacaa aaaaaaaaaa aaaaaaaa 1858

<210> 3
<211> 410
<212> PRT
<213> Mus musculus

<220>
<221> Angiopoietin-like protein 4 (ANGPTL4)
<222> (1)..(410)
<223> protein sequence (NP_065606, as of 25.03.2004),

of gene under UniGene ID Mm. 196189

<400> 3

Met Arg Cys Ala Pro Thr Ala Gly Ala Ala Leu Val Leu Cys Ala Ala
1               5                   10                  15

Thr Ala Gly Leu Leu Ser Ala Gln Gly Arg Pro Ala Gln Pro Glu Pro
            20                  25                  30

Pro Arg Phe Ala Ser Trp Asp Glu Met Asn Leu Leu Ala His Gly Leu

        35                  40                  45

Leu Gln Leu Gly His Gly Leu Arg Glu His Val Glu Arg Thr Arg Gly
    50                  55                  60

Gln Leu Gly Ala Leu Glu Arg Arg Met Ala Ala Cys Gly Asn Ala Cys
65                  70                  75                  80

Gln Gly Pro Lys Gly Lys Asp Ala Pro Phe Lys Asp Ser Glu Asp Arg
                85                  90                  95

Val Pro Glu Gly Gln Thr Pro Glu Thr Leu Gln Ser Leu Gln Thr Gln
            100                 105                 110

Leu Lys Ala Gln Asn Ser Lys Ile Gln Gln Leu Phe Gln Lys Val Ala
        115                 120                 125

Gln Gln Gln Arg Tyr Leu Ser Lys Gln Asn Leu Arg Ile Gln Asn Leu
    130                 135                 140

Gln Ser Gln Ile Asp Leu Leu Ala Pro Thr His Leu Asp Asn Gly Val
145                 150                 155                 160

Asp Lys Thr Ser Arg Gly Lys Lys Leu Ser Lys Met Thr Gln Leu Ile
                165                 170                 175

Gly Leu Thr Ser Asn Ala Thr His Leu His Arg Pro Ala Arg Asp Cys
            180                 185                 190

Gln Glu Leu Phe Gln Glu Gly Glu Arg His Ser Gly Leu Phe Gln Ile
        195                 200                 205

Gln Pro Leu Gly Ser Pro Pro Phe Leu Val Asn Cys Glu Met Thr Ser
    210                 215                 220

Asp Gly Gly Trp Thr Val Ile Gln Arg Arg Leu Asn Gly Ser Val Asp
225                 230                 235                 240

Phe Asn Gln Ser Trp Glu Ala Tyr Lys Asp Gly Phe Gly Asp Pro Gln
                245                 250                 255

Gly Glu Phe Trp Leu Gly Leu Glu Lys Met His Ser Ile Thr Gly Asp
            260                 265                 270

Arg Gly Ser Gln Leu Ala Val Gln Leu Gln Asp Trp Asp Gly Asn Ala
        275                 280                 285

Lys Leu Leu Gln Phe Pro Ile His Leu Gly Gly Glu Asp Thr Ala Tyr

290                 295                 300

Ser Leu Gln Leu Thr Glu Pro Thr Ala Asn Glu Leu Gly Ala Thr Asn
305                 310                 315                 320

Val Ser Pro Asn Gly Leu Ser Leu Pro Phe Ser Thr Trp Asp Gln Asp
                325                 330                 335

His Asp Leu Arg Gly Asp Leu Asn Cys Ala Lys Ser Leu Ser Gly Gly
            340                 345                 350

Trp Trp Phe Gly Thr Cys Ser His Ser Asn Leu Asn Gly Gln Tyr Phe
        355                 360                 365

His Ser Ile Pro Arg Gln Arg Gln Glu Arg Lys Lys Gly Ile Phe Trp
    370                 375                 380

Lys Thr Trp Lys Gly Arg Tyr Tyr Pro Leu Gln Ala Thr Thr Leu Leu
385                 390                 395                 400

Ile Gln Pro Met Glu Ala Thr Ala Ala Ser
                405                 410

<210>  4
<211>  21
<212>  DNA
<213>  Mus musculus

<220>
<221>  ANGPTL4 forward primer
<222>  (1)..(21)

<400>  4
tgctccaatt tcccatccaa t                                                21

<210>  5
<211>  21
<212>  DNA
<213>  Mus musculus

<220>
<221>  ANGPTL4 reverse primer
<222>  (1)..(21)

<400>  5
cagtgagctg caggctgtag g                                                21

<210>  6
<211>  21
<212>  DNA
<213>  Mus musculus

<220>
<221>  S12 forward primer
<222>  (1)..(21)

<400>  6
tgaaccagat gcaccgctta g                                                21


<210>  7
<211>  20
<212>  DNA
<213>  Mus musculus


<220>
<221>  S12 reverse primer
<222>  (1)..(20)

<400>  7
ttcttctttt gcacgtggcc                                                  20


<210>  8
<211>  624
<212>  DNA
<213>  Homo sapiens


<220>
<221>  human ANGPTL4 intron 3 containing four PPREs
<222>  (1)..(624)
<223>  segment of human chromosome 19 (GenBank: NT_077812), region from:
       8337915  to: 8338538

<400>  8
cacggtttct ctgtggtcct catccttccc tgcatctgtg gctgtccaca gccaactggg      60

tgaaagtttg gatccccctc tcacacccta gggtcagtgg caggcttcca gcactgtaga     120

cctgagggtt ctctcctccc caagctcccg ctccttccca cctccgtgct gcccgccccc     180

aaccccgcca ggctagcatc tcagcgtggt cagggtcctg tccaccctcc caaagccacc     240

atcccaggat gaggggcttc tggagggtga cgggggaagg cacaagtcct ggctgggaaa     300

tgcggtggaa gggggcaggg gttctgtggg ttcgggactc ccagactctt ggctcaggcc     360

cgccaagtag gagaaagttc agagctggga aggcgaacag ctggcattca tggaagccac     420

actggtggtt tggccgcgtg cccatcctta ctggatggga ggaaagtagg ggaaagggga     480

gatgcctgag gggccggaaa gcgtcttcct ggtcactctg ggcccgcccc cacccccacc     540

gtgcagactc atttcgacct ttcccctact tttccggctg ggctgggggc ggttcctccc     600

agtctggagc gtctgagcct ccag                                            624


<210>  9
<211>  594
<212>  DNA
<213>  Mus musculus

<220>
<221>  human ANGPTL4 intron 3 containing four PPREs
<222>  (1)..(594)
<223>  Segment of chromosome 17 (GenBank: NT_039649 as of 11.01.2005),
      region  from: 32271133  to: 32271726

<400>  9
gccttgacaa gtcctctaaa ttcaggaaga accaccccca gcccagccag ggaaagtagg      60

agaaaggtca ctgggggagg ggcatgcagg cactttctgc cagccagctc cgtctgcact     120

gccaggagtg ggggcagacc cagaaagatg ggaagtatac tttccaatcc ctcgggcagc     180

tagctccctt tcccctactt tcctctcatc caatcgggac aggcacactg ccaaaccacc     240

catgtggttc ccattagggc cagctgtttg tctctccagc tctgaacttt cctctacttg     300

gctaggctga gtctgggagt tccaaagcca caaaaccacc ccctcagctc ttctcttcca     360

ccacatcacc catccagaac ctatgcctgc cgaccctgga aaagctcatc catgaccctc     420

gccatgccta ggtacttgcc taagtaaaag ccaggggagg aattaagtct aaggaggatt     480

gggctgaaag ccagcaagca gccttagggt gtgaagggga tatccaaact ttcacctagt     540

tggctgtgga gagccacaga tgtcaagaag ggtgaacgaa taggcatgaa ctca           594

<210>  10
<211>  585
<212>  DNA
<213>  Canis familiaris

<220>
<221>  dog ANGPTL4 intron 3 comprising four PPREs
<222>  (1)..(585)
<223>  Segment of chromosome 20 (GenBank: NW_139889 as of 11.01.2005),
      region  from: 55810817  to: 55811401

<400>  10
cggtcctcct accccatcac accccagcct ggcatcagtg gctgcgtgca accaactggg      60

tgaaagttcg gattcccccc tttcacaccc tggggctggt tgctggctgt cagcccttta     120

gtcttgaggg ctcctcctcc cttcccctcc ctccaactcc ctcccctcct ccccattccc     180

aggccagtaa gggtccagta caccctccca aacctcctcc ctgggatcag accggcaggc     240

gcgggtctgg gtcggggatg tgggggtgcg gggggggtgc gtgggtttgg ggactcccag     300

acccggggct cagccctgcc aagtaggaga agggtcagag ctggagagac aaacagctgg     360

tgttaatgga agccacattg gtggtttggc ccggtgccgt ggggattgga agggaggaaa     420

gtaggggaaa ggggagctgc tccaggggcc ggaagaggtc tccagtcact ctgggtccgc     480

ccccctcccc ccagcgtgga cttcccctcc cccgggcgac ctttccaata ctttccccgg     540

ctgggctggc actggatgcg ggctttcatc agcagtttgg acgcg                     585

<210>  11

<211>  591
<212>  DNA
<213>  Rattus norvegicus


<220>
<221>  rat ANGPTL4 intron 3 comprising 4 PPREs
<222>  (1)..(591)
<223>  segment of rat chromosome 7 (GenBank: NW_047733 as of
       11.01.2005), region from 16265610 to:16266200

<400>  11
gccttgacaa gtcctctaaa ttcaggaaga accgccccca gcccagccag ggaaagtagg      60

agaaaggtca ctgggggagg agcatgcagg gactttctgc cagccagctc agtccacact     120

gccaggagtg ggggcagacc cagaaagatg ggaagtctac tttccaatcc cttgggcagc     180

tccctttccc ctactttcct ctcatccaat cgggacaggc acactgccaa gccacccatg     240

tggttttcat tagggccagc tgtttgtctc cccagctctg aactttcctc tacttggcta     300

ggctgagtct gggagtccca aacccacaaa accaccccct cagctcttct cttccaccac     360

atcacctatc cagaagctat gcctgccaac cctgagaaag ctcatccgtg accctcgaca     420

tgcctaggta cttgcctagg ttaaagccag gggacggatt aagtctaaag acaattgggc     480

tgacagccag caaccagcct tagggtgtga agggggatat ccaacctttc acctagttgg     540

ctgtgggtag ccacagatgt caagcagggt gagagaatag gcatgaactc a              591


<210>  12
<211>  23
<212>  DNA
<213>  Homo sapiens


<220>
<221>  hANGPTL4ClonF2
<222>  (1)..(23)
<223>  human ANGPTL4 intron 3 forward primer

<400>  12
ctcgcggttc tctaagttca cgg                                              23


<210>  13
<211>  24
<212>  DNA
<213>  Homo sapiens


<220>
<221>  hANGPTL4ClonR2
<222>  (1)..(23)
<223>  human ANGPTL4 intron 3 reverse primer

<400>  13
aggaggcagg gttgaggaaa gaaa                                             24


<210>  14

<211>  24
<212>  DNA
<213>  Mus musculus

<220>
<221>  mANGPTL4ClonF2
<222>  (1)..(24)
<223>  mouse ANGPTL4 intron 3 forward primer

<400>  14
cgttcaccct tcttgacatc tgtg                                              24

<210>  15
<211>  23
<212>  DNA
<213>  Mus musculus

<220>
<221>  mANGPTL4ClonR2
<222>  (1)..(23)
<223>  mouse ANGPTL4 intron 3 reverse primer

<400>  15
cttggccttg acaagtcctc taa                                               23

<210>  16
<211>  711
<212>  DNA
<213>  Homo sapiens

<220>
<221>  cloned hANGPTL4 control region
<222>  (1)..(711)

<400>  16
ctcgcggttc tctaagttca cggccccaca cggtttctct gtggtcctca tccttccctg      60

catctgtggc tgtccacagc caactgggtg aaagtttgga tccccctctc acaccctagg     120

gtcagtggca ggcttccagc actgtagacc tgagggttct ctcctcccca agctcccgct     180

ccttcccacc tccgtgctgc ccgcccccaa ccccgccagg ctagcatctc agcgtggtca     240

gggtcctgtc caccctccca aagccaccat cccaggatga ggggcttctg gagggtgacg     300

ggggaaggca caagtcctgg ctgggaaatg cggtggaagg gggcagggtt ctgtgggttc     360

gggactccca gactcttggc tcaggcccgc caagtaggag aaagttcaga gctgggaagg     420

cgaacagctg gcattcatgg aagccacact ggtggtttgg ccgcgtgccc atccttactg     480

gatgggagga aagtagggga aaggggagat gcctgagggg ccggaaagcg tcttcctggt     540

cactctgggc ccgcccccac ccccaccgtg cagactcatt tcgacctttc ccctactttt     600

ccggctgggc tgggggcggt tcctcccagt ctggagcgtc tgagcctcca gacgtgctca     660

acgccatcct cccctcctcc ctccctcttt ctttcctcaa ccctgcctcc t              711

<210>  17
<211>  582
<212>  DNA
<213>  Mus musculus

<220>
<221>  cloned mANGPTL4 control region
<222>  (1)..(582)

<400>  17
cgttcaccct tcttgacatc tgtggctctc cacagccaac taggtgaaag tttggatatc      60

cccttcacac cctaaggctg cttgctggct ttcagcccaa tcctccttag acttaattcc     120

tcccctggct tttacttagg caagtaccta ggcatggcga gggtcatgga tgagcttttc     180

cagggtcggc aggcataggt tctggatggg tgatgtggtg gaagagaaga gctgaggggg     240

tggttttgtg gctttggaac tcccagactc agcctagcca agtagaggaa agttcagagc     300

tggagagaca aacagctggc cctaatggga accacatggg tggtttggca gtgtgcctgt     360

cccgattgga tgagaggaaa gtaggggaaa gggagctagc tgcccgaggg attggaaagt     420

atacttccca tctttctggg tctgccccca ctcctggcag tgcagacgga gctggctggc     480

agaaagtgcc tgcatgcccc tcccccagtg acctttctcc tactttccct ggctgggctg     540

ggggtggttc ttcctgaatt tagaggactt gtcaaggcca ag                        582

<210>  18
<211>  28
<212>  DNA
<213>  Mus musculus

<220>
<221>  mANGPTL4 PPRE1 mutagenesis primer
<222>  (1)..(28)
<223>  Primer for deletion of 13 core DR1-encoding nucleotides of PPRE1
       of mouse ANGPTL4

<400>  18
ctccacagcc aactgatatc cccttcac                                         28

<210>  19
<211>  28
<212>  DNA
<213>  Mus musculus

<220>
<221>  mANGPTL4 PPRE2 mutagenesis primer
<222>  (1)..(28)
<223>  Primer for deletion of 13 core DR1-encoding nucleotides of PPRE2
       of mouse ANPTL4

<400>  19
cagcctagcc aagtgagctg gagagaca                                         28

<210>  20
<211>  28
<212>  DNA
<213>  Mus musculus


<220>
<221>  mANGPTL4 PPRE3 mutagenesis primer
<222>  (1)..(28)
<223>  primer for deletion of 13 core DR1-encoding nucleotides of PPRE3
       of mouse ANGPTL4

<400>  20
gatgagagga aagtctagct gcccgagg                                          28


<210>  21
<211>  28
<212>  DNA
<213>  Mus musculus


<220>
<221>  mANGPTL4 PPRE4 mutagenesis primer
<222>  (1)..(28)
<223>  primer for deletion of 13 core DR1-encoding nucleotides of PPRE4
       of mouse ANGPTL4

<400>  21
tgcccctccc ccagactttc cctggctg                                          28


<210>  22
<211>  22
<212>  DNA
<213>  Mus musculus


<220>
<221>  mANGPTL4-PPRE 1
<222>  (1)..(22)

<400>  22
gccaactagg tgaaagtttg ga                                                22


<210>  23
<211>  22
<212>  DNA
<213>  Rattus norvegicus


<220>
<221>  rANGPTL4-PPRE 1
<222>  (1)..(22)

<400>  23
gccaactagg tgaaaggttg ga                                                22


<210>  24
<211>  22
<212>  DNA

<213>  Canis familiaris

<220>
<221>  dANGPTL4-PPRE 1
<222>  (1)..(22)

<400>  24
accaactggg tgaaagttcg ga                                                22

<210>  25
<211>  22
<212>  DNA
<213>  Homo sapiens

<220>
<221>  hANGPTL4-PPRE 1
<222>  (1)..(22)

<400>  25
gccaactggg tgaaagtttg ga                                                22

<210>  26
<211>  22
<212>  DNA
<213>  Mus musculus

<220>
<221>  mANGPTL4-PPRE 2
<222>  (1)..(22)

<400>  26
gccaagtaga ggaaagttca ga                                                22

<210>  27
<211>  22
<212>  DNA
<213>  Rattus norvegicus

<220>
<221>  rANGPTL4-PPRE 2
<222>  (1)..(22)

<400>  27
gccaagtaga ggaaagttca ga                                                22

<210>  28
<211>  22
<212>  DNA
<213>  Canis familiaris

<220>
<221>  dANGPTL4-PPRE 2
<222>  (1)..(22)

<400>  28

gccaagtagg agaagggtca ga                                                22

<210>  29
<211>  22
<212>  DNA
<213>  Homo sapiens

<220>
<221>  hANGPTL4-PPRE 2
<222>  (1)..(22)

<400>  29
gccaagtagg agaaagttca ga                                                22

<210>  30
<211>  22
<212>  DNA
<213>  Mus musculus

<220>
<221>  mANGPTL4-PPRE 3
<222>  (1)..(22)

<400>  30
ggaaagtagg ggaaagggag ct                                                22

<210>  31
<211>  22
<212>  DNA
<213>  Rattus norvegicus

<220>
<221>  rANGPTL4-PPRE 3
<222>  (1)..(22)

<400>  31
ggaaagtagg ggaaagggag ct                                                22

<210>  32
<211>  22
<212>  DNA
<213>  Canis familiaris

<220>
<221>  dANGPTL4-PPRE 3
<222>  (1)..(22)

<400>  32
ggaaagtagg ggaaagggga gc                                                22

<210>  33
<211>  22
<212>  DNA
<213>  Homo sapiens

<220>
<221>  hANGPTL4-PPRE 3
<222>  (1)..(22)

<400>  33
ggaaagtagg ggaaagggga ga                                                22

<210>  34
<211>  22
<212>  DNA
<213>  Mus musculus

<220>
<221>  mANGPTL4-PPRE 4
<222>  (1)..(22)

<400>  34
ggaaagtagg agaaaggtca ct                                                22

<210>  35
<211>  22
<212>  DNA
<213>  Rattus norvegicus

<220>
<221>  rANGPTL4-PPRE 4
<222>  (1)..(22)

<400>  35
ggaaagtagg agaaaggtca ct                                                22

<210>  36
<211>  22
<212>  DNA
<213>  Canis familiaris

<220>
<221>  dANGPTL4-PPRE 4
<222>  (1)..(22)

<400>  36
ggaaagtatt ggaaaggtcg cc                                                22

<210>  37
<211>  22
<212>  DNA
<213>  Homo sapiens

<220>
<221>  hANGPTL4-PPRE 4
<222>  (1)..(22)

<400>  37
gaaaagtagg ggaaaggtcg aa                                                22

<210>  38
<211>  17
<212>  DNA
<213>  Artificial

<220>
<223>  Description of artificial sequence: Consensus sequence of PPRE
       sequences of mouse, rat, dog and human


<220>
<221>  Consensus PPRE
<222>  (1)..(17)

<220>
<221>  misc_feature
<222>  (8)..(8)
<223>  n is a, c, g, or t

<400>  38
aactaggnca aaggtca                                                    17


<210>  39
<211>  96
<212>  DNA
<213>  herpes simplex virus 7


<220>
<221>  minimal promoter region from the herpes simplex thymidine kinase
       gene (pTK37)
<222>  (1)..(96)

<400>  39
gaattcggtc cacttcgcat attaaggtga cgcgtgtggc ctcgaacacc gagcgaccct     60

gcagcgaccc gcttaacagc gtcaacagcg tgccgc                               96

### Annex to the application documents - subsequently filed sequences listing

SEQUENCE LISTING

<110>  F. Hoffmann-La Roche

<120>  ANGPTL4/FIAF as Marker for PPAR modulation

<130>  22517

<140>  05006565.5
<141>  2005-03-24

<160>  39

<170>  PatentIn version 3.3

<210>  1
<211>  1883
<212>  DNA
<213>  Mus musculus


<220>
<221>  mRNA
<222>  (1)..(1870)
<223>  Angiopoietin-like protein 4 (ANGPTL4), representative cDNA
       (complete cds) (BC021343 as of 25.03.2004), for gene under
       UniGene ID Mm.196189

<400>  1
ccacgcgtcc ggctacgggc tccagatctt cttctgcacc agagcaagtc taagtctgag      60

ccggctcccc cagaactcca gctgctgggt cttgaactcc tgcgttccgg agtcctagcg     120

ttgctgcacc caaggccacc cccagaatca tgcgctgcgc tccgacagca ggcgctgccc     180

tggtgctatg cgcggctact gcggggcttt tgagcgcgca agggcgccct gcacagccag     240

agccaccgcg ctttgcatcc tgggacgaga tgaacttgct ggctcacggg ctgctacagc     300

tcggccatgg gctgcgcgaa cacgtggagc gcacccgtgg gcagctgggc gcgctggagc     360

gccgcatggc tgcctgtggt aacgcttgtc aggggcccaa gggaaaagat gcacccttca     420

aagactccga ggatagagtc cctgaaggcc agactcctga gactctgcag agtttgcaga     480

ctcagctcaa ggctcaaaac agcaagatcc agcaattgtt ccagaaggtg gcccagcagc     540

agagatacct atcaaagcag aatctgagaa tacagaatct tcagagccag atagacctct     600

tggcccccac gcacctagac aatggagtag acaagacttc gaggggaaag aggcttccca     660

agatgaccca gctcattggc ttgactccca acgccaccca cttacacagg ccgccccggg     720

actgccagga actcttccaa gaaggggaga ggcacagtgg acttttccag atccagcctc     780

tggggtctcc accatttttg gtcaactgtg agatgacttc agatggaggc tggacagtga     840

ttcagagacg cctgaacggc tctgtggact tcaaccagtc ctgggaagcc tacaaggatg     900

gcttcggaga tccccaaggc gagttctggc tgggcctgga aaagatgcac agcatcacag     960

ggaaccgagg aagccaattg gctgtgcagc tccaggactg ggatggcaat gccaaattgc    1020

tccaatttcc catccatttg gggggtgagg acacagccta cagcctgcag ctcactgagc    1080

ccacggccaa tgagctgggt gccaccaatg tttcccccaa tggcctttcc ctgcccttct    1140

ctacttggga ccaagaccat gacctccgtg gggaccttaa ctgtgccaag agcctctctg    1200

gtggctggtg gtttggtacc tgtagccatt ccaatctcaa tggacaatac ttccactcta 1260

tcccacggca acggcaggag cgtaaaaagg gtatcttctg gaaaacatgg aagggccgct 1320

actatcctct gcaggctacc accctgctga tccagcccat ggaggctaca gcagcctctt 1380

agcctcctca ctggagcctg gttccaggcc taagaagaca gtgactttgg ttgtggccct 1440

gagatttggc cattctctgc tgggggcagg agctctaagt agggctatct gcgtcttgtg 1500

gacaaagaag aagcccgtaa ctggagagac tggaggaccc cttttccgtg ttggggtctg 1560

caagcattgt tgtctgaaac agtcagagca acaggaaaca aatggcccag atccagaaaa 1620

catgggctcg aggggcactg aatatcactt ctcgcctacc agagaagttg gggatgcaga 1680

gggaccacta cagtccaact agctgggccc ttaatggcgg actcagtcat attgactgac 1740

tggagacagg gtgccaggag ccctggatac actcatggtg ctgttgtagg tgctgtggat 1800

gcacaggtgc taactgtggt tcccaggcac aactcacagc attcttacaa taaaaacaac 1860

ctcagaacaa aaaaaaaaaa aaa 1883

<210> 2
<211> 1858
<212> DNA
<213> Mus musculus

<220>
<221> mRNA
<222> (1)..(1858)
<223> Angiopoietin-like protein 4 (ANGPTL4), representative cDNA
(NM_020581, as of 25.03.2004), for gene under UniGene ID
Mm.196189

<400> 2
gcaccagagc aagtctaagt ctgagccggc tcccccagaa ctccagctgc tgggtcttga 60

actcctgcgt tccggagtcc tagcgttgct gcacccaagg ccacccccag aatcatgcgc 120

tgcgctccga cagcaggcgc tgccctggtg ctatgcgcgg ctactgcggg gcttttgagc 180

gctcaagggc gccctgcaca gccagagcca ccgcgctttg catcctggga cgagatgaac 240

ttgctggctc acgggctgct acagctcggc catgggctgc gcgaacacgt ggagcgcacc 300

cgtgggcagc tgggcgcgct ggagcgccgc atggctgcct gtggtaacgc ttgtcagggg 360

cccaagggaa aagatgcacc cttcaaagac tccgaggata gagtccctga aggccagact 420

cctgagactc tgcagagttt gcagactcag ctcaaggctc aaaacagcaa gatccagcaa 480

ttgttccaga aggtggccca gcagcagaga tacctatcaa agcagaatct gagaatacag 540

aatcttcaga gccagataga cctcttggcc cccacgcacc tggacaatgg agtagacaag 600

acttcgaggg gaaagaagct ttccaagatg acccagctca ttggcttgac ttccaacgcc 660

acccacttac acaggccggc ccgggactgc caggaactct tccaagaagg ggagaggcac 720

agtggacttt tccagatcca gcctctgggg tctccaccat ttttggtcaa ctgtgagatg 780

acttcagatg gaggctggac agtgattcag agacgcctga acggctctgt ggacttcaac 840

cagtcctggg aagcctacaa ggatggcttc ggagatcccc aaggcgagtt ctggctgggc 900

ctggaaaaga tgcacagcat cacaggggac cgaggaagcc aattggctgt gcagctccag 960

gactgggatg gcaatgccaa attgctccaa tttcccatcc atttgggggg tgaggacaca 1020

gcctacagcc tgcagctcac tgagcccacg gccaatgagc tgggtgccac caatgtttcc 1080

cccaatggcc tttccctgcc cttctctact tgggaccaag accatgacct ccgtggggac 1140

cttaactgtg ccaagagcct ctctggtggc tggtggtttg gtacctgtag ccattccaat 1200

ctcaatggac aatacttcca ctctatccca cggcaacggc aggagcgtaa aaagggtatc 1260

ttctggaaaa catggaaggg ccgctactat cctctgcagg ctaccaccct gttgatccag 1320

cccatggagg ctacagcagc ctcttagcct cctcactgga gcctggttcc aggcctaaga 1380

agacagtgac tttggttgtg gccctgagat ttggccattc tctgctgggg gcaggagctc 1440

taagtagggc tatctgcgtc ttgtggacaa agaagaagcc cgtaactgga gagactggag 1500

gacccctttt ccgtgttggg gtctgcaagc attgttgtct gaaacagtca gagcaacagg 1560

aaacaaatgg cccagatcca gaaaacatgg gctcgagggg cactgaatat cacttctcgc 1620

ctaccagaga agttggggat gcagagggac cactacagtc caactagctg ggcccttaat 1680

ggcggactca gtcatattga ctgactggag acagggtgcc aggagccctg gatacactca 1740

tggtgctgtt gtaggtgctg tggatgcaca ggtgctaact gtggttccca ggcacagctc 1800

acagcattct tacaataaaa acaacctcag aacaaaacaa aaaaaaaaaa aaaaaaaa 1858

<210> 3
<211> 410
<212> PRT
<213> Mus musculus

<220>
<221> CHAIN
<222> (1)..(410)
<223> Angiopoietin-like protein 4 (ANGPTL4), protein sequence
(NP_065606, as of 25.03.2004), of gene under UniGene ID Mm.
196189

<400> 3

Met Arg Cys Ala Pro Thr Ala Gly Ala Ala Leu Val Leu Cys Ala Ala
1               5                   10                  15

Thr Ala Gly Leu Leu Ser Ala Gln Gly Arg Pro Ala Gln Pro Glu Pro
            20                  25                  30

Pro Arg Phe Ala Ser Trp Asp Glu Met Asn Leu Leu Ala His Gly Leu
        35                  40                  45

Leu Gln Leu Gly His Gly Leu Arg Glu His Val Glu Arg Thr Arg Gly
    50                  55                  60

Gln Leu Gly Ala Leu Glu Arg Arg Met Ala Ala Cys Gly Asn Ala Cys
65                  70                  75                  80

Gln Gly Pro Lys Gly Lys Asp Ala Pro Phe Lys Asp Ser Glu Asp Arg
                85                  90                  95

Val Pro Glu Gly Gln Thr Pro Glu Thr Leu Gln Ser Leu Gln Thr Gln
            100                 105                 110

Leu Lys Ala Gln Asn Ser Lys Ile Gln Gln Leu Phe Gln Lys Val Ala
        115                 120                 125

Gln Gln Gln Arg Tyr Leu Ser Lys Gln Asn Leu Arg Ile Gln Asn Leu
    130                 135                 140

Gln Ser Gln Ile Asp Leu Leu Ala Pro Thr His Leu Asp Asn Gly Val
145                 150                 155                 160

Asp Lys Thr Ser Arg Gly Lys Lys Leu Ser Lys Met Thr Gln Leu Ile
                165                 170                 175

Gly Leu Thr Ser Asn Ala Thr His Leu His Arg Pro Ala Arg Asp Cys
            180                 185                 190

Gln Glu Leu Phe Gln Glu Gly Glu Arg His Ser Gly Leu Phe Gln Ile
        195                 200                 205

Gln Pro Leu Gly Ser Pro Pro Phe Leu Val Asn Cys Glu Met Thr Ser
    210                 215                 220

Asp Gly Gly Trp Thr Val Ile Gln Arg Arg Leu Asn Gly Ser Val Asp
225                 230                 235                 240

Phe Asn Gln Ser Trp Glu Ala Tyr Lys Asp Gly Phe Gly Asp Pro Gln
                245                 250                 255

Gly Glu Phe Trp Leu Gly Leu Glu Lys Met His Ser Ile Thr Gly Asp
            260                 265                 270

Arg Gly Ser Gln Leu Ala Val Gln Leu Gln Asp Trp Asp Gly Asn Ala
        275                 280                 285

Lys Leu Leu Gln Phe Pro Ile His Leu Gly Gly Glu Asp Thr Ala Tyr
    290                 295                 300

Ser Leu Gln Leu Thr Glu Pro Thr Ala Asn Glu Leu Gly Ala Thr Asn
305                 310                 315                 320

Val Ser Pro Asn Gly Leu Ser Leu Pro Phe Ser Thr Trp Asp Gln Asp
                325                 330                 335

His Asp Leu Arg Gly Asp Leu Asn Cys Ala Lys Ser Leu Ser Gly Gly
            340                 345                 350

Trp Trp Phe Gly Thr Cys Ser His Ser Asn Leu Asn Gly Gln Tyr Phe
        355                 360                 365

His Ser Ile Pro Arg Gln Arg Gln Glu Arg Lys Lys Gly Ile Phe Trp
    370                 375                 380

Lys Thr Trp Lys Gly Arg Tyr Tyr Pro Leu Gln Ala Thr Thr Leu Leu
385                 390                 395                 400

Ile Gln Pro Met Glu Ala Thr Ala Ala Ser
                405                 410

<210>  4
<211>  21
<212>  DNA
<213>  Mus musculus

<400>  4
tgctccaatt tcccatccaa t                                                21

<210>  5
<211>  21
<212>  DNA
<213>  Mus musculus

<400>  5
cagtgagctg caggctgtag g                                                21

<210>  6
<211>  21
<212>  DNA
<213>  Mus musculus

<400>  6
tgaaccagat gcaccgctta g                                                21

<210>  7
<211>  20
<212>  DNA
<213>  Mus musculus

<400>  7
ttcttctttt gcacgtggcc                                                  20

<210>  8
<211>  624
<212>  DNA
<213>  Homo sapiens

<220>
<221>  Intron
<222>  (1)..(624)
<223>  human ANGPTL4 intron 3 containing four PPREs, segment of human
       chromosome 19 (GenBank: NT_077812), region from: 8337915  to:
       8338538

<400>  8

cacggtttct ctgtggtcct catccttccc tgcatctgtg gctgtccaca gccaactggg 60

tgaaagtttg gatccccctc tcacacccta gggtcagtgg caggcttcca gcactgtaga 120

cctgagggtt ctctcctccc caagctcccg ctccttccca cctccgtgct gcccgccccc 180

aaccccgcca ggctagcatc tcagcgtggt cagggtcctg tccaccctcc caaagccacc 240

atcccaggat gaggggcttc tggagggtga cgggggaagg cacaagtcct ggctgggaaa 300

tgcggtggaa gggggcaggg gttctgtggg ttcgggactc ccagactctt ggctcaggcc 360

cgccaagtag gagaaagttc agagctggga aggcgaacag ctggcattca tggaagccac 420

actggtggtt tggccgcgtg cccatcctta ctggatggga ggaaagtagg ggaaagggga 480

gatgcctgag gggccggaaa gcgtcttcct ggtcactctg ggcccgcccc cacccccacc 540

gtgcagactc atttcgacct ttcccctact tttccggctg ggctgggggc ggttcctccc 600

agtctggagc gtctgagcct ccag 624

<210> 9
<211> 594
<212> DNA
<213> Mus musculus

<220>
<221> Intron
<222> (1)..(594)
<223> mouse ANGPTL4 intron 3 containing four PPREs, Segment of
chromosome 17 (GenBank: NT_039649 as of 11.01.2005), region
from: 32271133 to: 32271726

<400> 9
gccttgacaa gtcctctaaa ttcaggaaga accaccccca gcccagccag ggaaagtagg 60

agaaaggtca ctgggggagg ggcatgcagg cactttctgc cagccagctc cgtctgcact 120

gccaggagtg ggggcagacc cagaaagatg ggaagtatac tttccaatcc ctcgggcagc 180

tagctccctt tcccctactt tcctctcatc caatcgggac aggcacactg ccaaaccacc 240

catgtggttc ccattagggc cagctgtttg tctctccagc tctgaacttt cctctacttg 300

gctaggctga gtctgggagt tccaaagcca caaaaccacc ccctcagctc ttctcttcca 360

ccacatcacc catccagaac ctatgcctgc cgaccctgga aaagctcatc catgaccctc 420

gccatgccta ggtacttgcc taagtaaaag ccaggggagg aattaagtct aaggaggatt 480

gggctgaaag ccagcaagca gccttagggt gtgaagggga tatccaaact ttcacctagt 540

tggctgtgga gagccacaga tgtcaagaag ggtgaacgaa taggcatgaa ctca 594

<210> 10
<211> 585
<212> DNA
<213> Canis familiaris

<220>
<221> Intron
<222> (1)..(585)
<223> dog ANGPTL4 intron 3 comprising four PPREs, Segment of chromosome

20 (GenBank: NW_139889 as of 11.01.2005), region  from: 55810817
to: 55811401

<400>  10
cggtcctcct accccatcac accccagcct ggcatcagtg gctgcgtgca accaactggg      60

tgaaagttcg gattcccccc tttcacaccc tggggctggt tgctggctgt cagcccttta     120

gtcttgaggg ctcctcctcc cttcccctcc ctccaactcc ctcccctcct ccccattccc     180

aggccagtaa gggtccagta caccctccca aacctcctcc ctgggatcag accggcaggc     240

gcgggtctgg gtcggggatg tgggggtgcg gggggggtgc gtgggtttgg ggactcccag     300

acccggggct cagccctgcc aagtaggaga agggtcagag ctggagagac aaacagctgg     360

tgttaatgga agccacattg gtggtttggc ccggtgccgt ggggattgga agggaggaaa     420

gtaggggaaa ggggagctgc tccaggggcc ggaagaggtc tccagtcact ctgggtccgc     480

ccccctcccc ccagcgtgga cttcccctcc cccgggcgac ctttccaata ctttccccgg     540

ctgggctggc actggatgcg ggctttcatc agcagtttgg acgcg                     585

<210>  11
<211>  591
<212>  DNA
<213>  Rattus norvegicus

<220>
<221>  Intron
<222>  (1)..(591)
<223>  rat ANGPTL4 intron 3 comprising 4 PPREs, segment of rat
       chromosome 7 (GenBank: NW_047733 as of 11.01.2005), region from
       16265610 to:16266200

<400>  11
gccttgacaa gtcctctaaa ttcaggaaga accgccccca gcccagccag ggaaagtagg      60

agaaaggtca ctgggggagg agcatgcagg gactttctgc cagccagctc agtccacact     120

gccaggagtg ggggcagacc cagaaagatg ggaagtctac tttccaatcc cttgggcagc     180

tccctttccc ctactttcct ctcatccaat cgggacaggc acactgccaa gccacccatg     240

tggttttcat tagggccagc tgtttgtctc cccagctctg aactttcctc tacttggcta     300

ggctgagtct gggagtccca aacccacaaa accaccccct cagctcttct cttccaccac     360

atcacctatc cagaagctat gcctgccaac cctgagaaag ctcatccgtg accctcgaca     420

tgcctaggta cttgcctagg ttaaagccag gggacggatt aagtctaaag acaattgggc     480

tgacagccag caaccagcct tagggtgtga agggggatat ccaacctttc acctagttgg     540

ctgtgggtag ccacagatgt caagcagggt gagagaatag gcatgaactc a              591

<210>  12
<211>  23
<212>  DNA
<213>  Homo sapiens

<400>  12
ctcgcggttc tctaagttca cgg                                              23

<210>  13
<211>  24
<212>  DNA
<213>  Homo sapiens

<400>  13
aggaggcagg gttgaggaaa gaaa                                              24


<210>  14
<211>  24
<212>  DNA
<213>  Mus musculus

<400>  14
cgttcaccct tcttgacatc tgtg                                              24


<210>  15
<211>  23
<212>  DNA
<213>  Mus musculus

<400>  15
cttggccttg acaagtcctc taa                                               23


<210>  16
<211>  711
<212>  DNA
<213>  Homo sapiens

<400>  16
ctcgcggttc tctaagttca cggccccaca cggtttctct gtggtcctca tccttccctg      60

catctgtggc tgtccacagc caactgggtg aaagtttgga tccccctctc acaccctagg     120

gtcagtggca ggcttccagc actgtagacc tgagggttct ctcctcccca agctcccgct     180

ccttcccacc tccgtgctgc ccgcccccaa ccccgccagg ctagcatctc agcgtggtca     240

gggtcctgtc caccctccca aagccaccat cccaggatga ggggcttctg gagggtgacg     300

ggggaaggca caagtcctgg ctgggaaatg cggtggaagg gggcagggtt ctgtgggttc     360

gggactccca gactcttggc tcaggcccgc caagtaggag aaagttcaga gctgggaagg     420

cgaacagctg gcattcatgg aagccacact ggtggtttgg ccgcgtgccc atccttactg     480

gatgggagga aagtagggga aaggggagat gcctgagggg ccggaaagcg tcttcctggt     540

cactctgggc ccgcccccac ccccaccgtg cagactcatt tcgacctttc ccctactttt     600

ccggctgggc tgggggcggt tcctcccagt ctggagcgtc tgagcctcca gacgtgctca     660

acgccatcct cccctcctcc ctccctcttt ctttcctcaa ccctgcctcc t              711


<210>  17
<211>  582
<212>  DNA
<213>  Mus musculus

<400>  17
cgttcaccct tcttgacatc tgtggctctc cacagccaac taggtgaaag tttggatatc      60

cccttcacac cctaaggctg cttgctggct ttcagcccaa tcctccttag acttaattcc     120

tcccctggct tttacttagg caagtaccta ggcatggcga gggtcatgga tgagcttttc    180

cagggtcggc aggcataggt tctggatggg tgatgtggtg gaagagaaga gctgaggggg    240

tggttttgtg gctttggaac tcccagactc agcctagcca agtagaggaa agttcagagc    300

tggagagaca aacagctggc cctaatggga accacatggg tggtttggca gtgtgcctgt    360

cccgattgga tgagaggaaa gtaggggaaa gggagctagc tgcccgaggg attggaaagt    420

atacttccca tctttctggg tctgccccca ctcctggcag tgcagacgga gctggctggc    480

agaaagtgcc tgcatgcccc tcccccagtg acctttctcc tactttccct ggctgggctg    540

ggggtggttc ttcctgaatt tagaggactt gtcaaggcca ag                        582

<210>  18
<211>  28
<212>  DNA
<213>  Mus musculus

<400>  18
ctccacagcc aactgatatc cccttcac                                         28

<210>  19
<211>  28
<212>  DNA
<213>  Mus musculus

<400>  19
cagcctagcc aagtgagctg gagagaca                                         28

<210>  20
<211>  28
<212>  DNA
<213>  Mus musculus

<400>  20
gatgagagga aagtctagct gcccgagg                                         28

<210>  21
<211>  28
<212>  DNA
<213>  Mus musculus

<400>  21
tgcccctccc ccagactttc cctggctg                                         28

<210>  22
<211>  22
<212>  DNA
<213>  Mus musculus

<400>  22
gccaactagg tgaaagtttg ga                                               22

<210>  23
<211>  22
<212>  DNA
<213>  Rattus norvegicus

<400>  23
gccaactagg tgaaaggttg ga                                                22

<210>  24
<211>  22
<212>  DNA
<213>  Canis familiaris

<400>  24
accaactggg tgaaagttcg ga                                                22

<210>  25
<211>  22
<212>  DNA
<213>  Homo sapiens

<400>  25
gccaactggg tgaaagtttg ga                                                22

<210>  26
<211>  22
<212>  DNA
<213>  Mus musculus

<400>  26
gccaagtaga ggaaagttca ga                                                22

<210>  27
<211>  22
<212>  DNA
<213>  Rattus norvegicus

<400>  27
gccaagtaga ggaaagttca ga                                                22

<210>  28
<211>  22
<212>  DNA
<213>  Canis familiaris

<400>  28
gccaagtagg agaagggtca ga                                                22

<210>  29
<211>  22
<212>  DNA
<213>  Homo sapiens

<400>  29
gccaagtagg agaaagttca ga                                                22

<210>  30
<211>  22
<212>  DNA
<213>  Mus musculus

<400>  30
ggaaagtagg ggaaagggag ct                                                22

<210>  31

<211>  22
<212>  DNA
<213>  Rattus norvegicus

<400>  31
ggaaagtagg ggaaagggag ct                                                22


<210>  32
<211>  22
<212>  DNA
<213>  Canis familiaris

<400>  32
ggaaagtagg ggaaagggga gc                                                22


<210>  33
<211>  22
<212>  DNA
<213>  Homo sapiens

<400>  33
ggaaagtagg ggaaagggga ga                                                22


<210>  34
<211>  22
<212>  DNA
<213>  Mus musculus

<400>  34
ggaaagtagg agaaaggtca ct                                                22


<210>  35
<211>  22
<212>  DNA
<213>  Rattus norvegicus

<400>  35
ggaaagtagg agaaaggtca ct                                                22


<210>  36
<211>  22
<212>  DNA
<213>  Canis familiaris

<400>  36
ggaaagtatt ggaaaggtcg cc                                                22


<210>  37
<211>  22
<212>  DNA
<213>  Homo sapiens

<400>  37
gaaaagtagg ggaaaggtcg aa                                                22


<210>  38
<211>  17
<212>  DNA
<213>  Artificial

<220>

<223>  Description of artificial sequence: Consensus sequence of PPRE
      sequences of mouse, rat, dog and human

<220>
<221>  misc_feature
<222>  (8)..(8)
<223>  n is a, c, g, or t

<400>  38
aactaggnca aaggtca                                                    17

<210>  39
<211>  96
<212>  DNA
<213>  herpes simplex virus 7

<400>  39
gaattcggtc cacttcgcat attaaggtga cgcgtgtggc ctcgaacacc gagcgaccct     60

gcagcgaccc gcttaacagc gtcaacagcg tgccgc                               96

## Description

Peroxisome Proliferator Activated Receptors (PPARs) are members of the nuclear hormone receptor superfamily. The PPARs are ligand-activated transcription factors that regulate gene expression and control multiple metabolic pathways. Three subtypes have been described which are PPARα, PPARδ (also known as PPARβ), and PPARγ. PPARδ is ubiquitously expressed. PPARα is predominantly expressed in the liver, kidney and heart. There are at least two major isoforms of PPARγ. PPARγ1 is expressed in most tissues, and the longer isoform, PPARγ2 is almost exclusively expressed in adipose tissue. The PPARs modulate a variety of physiological responses including regulation of glucose- and lipid-homeostasis and metabolism, energy balance, cell differentiation, inflammation and cardiovascular events.

Approximately half of all patients with coronary artery disease have low concentrations of plasma High Density Lipidprotein Cholesterol (HDL-C). The atheroprotective function of HDL was first highlighted almost 25 years ago and stimulated exploration of the genetic and environmental factors that influence HDL levels. The protective function of HDL comes from its role in a process termed reverse cholesterol transport. HDL mediates the removal of cholesterol from cells in peripheral tissues including those in the atherosclerotic lesions of the arterial wall. HDL then delivers its cholesterol to the liver and sterol-metabolizing organs for conversion to bile and elimination. Data from the Framingham study showed that HDL-C levels are predictive of coronary artery disease risk independently of LDL-C (Low Density Lipidprotein Cholesterol) levels (Gordon et al., *Am. J. Med.* 1977, 62, 707-714). The estimated age-adjusted prevalence among Americans age 20 and older who have HDL-C of less than 35 mg/dl is 16% (males) and 5.7 % (females). A substantial increase of HDL-C is currently achieved by treatment with niacin in various formulations. However, the substantial side-effects limit the therapeutic potential of this approach.

As many as 90% of the 14 million diagnosed type 2 diabetic patients in the US are overweight or obese, and a high proportion of type 2 diabetic patients have abnormal concentrations of lipoproteins. The prevalence of total cholesterol > 240 mg/dl is 37% in diabetic men and 44% in women. The respective rates for LDL-C > 160 mg/dl are 31% and 44%, respectively, and for HDL-C < 35 mg/dl 28% and 11%, respectively. Diabetes is a disease in which a patient's ability to control glucose levels in blood is decreased because of partial impairment in response to the action of insulin. Type II diabetes (T2D) is also called non-insulin dependent diabetes mellitus (NIDDM) and afflicts 80-90 % of all diabetic patients in developed countries. In T2D, the pancreatic Islets of Langerhans continue to produce insulin. However, the target organs for insulin action, mainly muscle, liver and adipose tissue, exhibit a profound resistance to insulin stimulation. The body continues to compensate by producing unphysiologically high levels of insulin, which ultimately decreases in later stage of disease, due to exhaustion and failure of pancreatic insulin-producing capacity. Thus T2D is a cardiovascular-metabolic syndrome associated with multiple comorbidities including insulin resistance, dyslipidemia, hypertension, endothelial dysfunction and inflammatory atherosclerosis.

First line treatment for dyslipidemia and diabetes generally involves a low-fat and low-glucose diet, exercise and weight loss. However, compliance can be moderate, and as the disease progresses, treatment of the various metabolic deficiencies becomes necessary with e.g. lipid-modulating agents such as statins and fibrates for dyslipidemia and hypoglycemic drugs, e.g. sulfonylureas or metformin for insulin resistance. A promising new class of drugs has recently been introduced that resensitizes patients to their own insulin (insulin sensitizers), thereby restoring blood glucose and triglyceride levels to normal, and in many cases, obviating or reducing the requirement for exogenous insulin. Pioglitazone (Actos™) and rosiglitazone (Avandia™) belong to the thiazolidinedione (TZD) class of PPARγ-agonists and were the first in their class to be approved for NIDDM in several countries. These compounds, however, suffer from side effects, including rare but severe liver toxicity (as seen with troglitazone). They also increase body weight in patients. Therefore, new, more efficacious drugs with greater safety and lower incidence of side effects are urgently needed. Recent studies provide evidence that agonism of PPARδ would result in compounds with enhanced therapeutic potential, i. e. such compounds should improve the lipid profile, with a superior effect on HDL-C rising compared to current treatments and with additional positive effects on normalization of insulin-levels (Oliver et al; Proc Nat Acad Sci USA 2001; 98: 5306-11). Recent observations also suggest that there is an independent PPARα mediated effect on insulin-sensitization in addition to its well known role in reducing triglycerides (Guerre-Millo et al; J Biol Chem 2000; 275: 16638-16642). Thus, selective PPARδ agonists or PPARδ agonists with additional PPARα activity may show superior therapeutic efficacy without the side-effects such as the weight gain seen with PPARγ agonists.

The present invention relates to a marker for PPARdelta modulation, and methods of diagnosing a disease linked to dysregulation of PPARdelta activity, methods of monitoring the treatment of patients suffering from a disease linked to dysregulation of PPARdelta activity, and methods of identifying compounds which modulate PPARdelta activity.

The gene encoding Angiopoietin-like protein 4 (ANGL-4, ANGPTL4), also known as Fasting Induced Adipose Factor (FIAF), PPARgamma Angiopoietin Related protein (PGAR) and Hepatic Fibrinogen/Angiopoietin-Related Protein (HFARP), was identified as direct PPARdelta target gene and this gene may be used as biomarker for PPARdelta.

ANGPTL4 is already described as target gene of PPARalpha and of PPARgamma. The mRNA expression of ANGPTL4 is stimulated by PPARalpha in the liver, whereas PPARgamma stimulates a selective expression of ANGPTL4 in highly vascular tissues such as fat and placenta. This is consistent with the implication of ANGPTL4 in the metabolic response to fasting. (Kersten et al., *J. Biol. Chem.* **2000**, 275, 28488-28493; Yoon et al., *Mol. Cell. Biol.* **2000**, 20, 5343-5349)

However, the present invention provides that the contribution of PPARdelta on ANGPTL4 gene is in certain tissues and cells much larger than of PPARalpha or PPARgamma. Particularly in muscle cells the ANGPTL4 gene is mainly activated by PPARdelta. Therefore, the present invention permits to distinguish the response elicited by PPARdelta from the response elicited by PPARalpha.

The present invention provides the use of ANGPTL4 as marker for detecting or monitoring PPARdelta modulation. In a preferred embodiment ANGPTL4 is used as marker for detecting or monitoring PPARdelta modulation in muscle.

The term "modulation" as used herein relates to an activation or inhibition of the transcriptional activity of PPARdelta. Thus, ANGPTL4 can serve as marker for modulation of PPARdelta activity.

The term "marker" as used herein refers to nucleic acid or polypeptide.

The present invention also pertains to ANGPTL4 as marker for diagnosing a disease involving dysregulation of PPARdelta activity such as for example dyslipidemia, obesity or insulin resistance. Thus, ANGPTL4 can serve as marker detecting for the modulation of PPARdelta activity.

The present invention further provides a method of detecting or monitoring the activity of PPARdelta in a host comprising quantifying the expression level of ANGPTL4 mRNA. A representative cDNA of mouse ANGPTL4 is shown in SEQ. ID NO: 1.
In one embodiment of the method hereinbefore described the mRNA expression level of ANGPTL4 is determined relative to a control.

A host may be an animal, tissue, cells or any other biological system that is capable of RNA transcription including in vitro transcription. Preferably, the animal is a non-human animal. The control may be the level of mRNA expression of ANGPTL4 in a different host.

Furthermore, the present invention provides a method of determining whether a test compound modulates PPARdelta activity in a host comprising
a) exposing the host to the test compound and
b) quantifying the mRNA expression level of ANGPTL4.
In one embodiment of the method hereinbefore described the mRNA expression level of ANGPTL4 is determined relative to a control.

A host may be an animal, tissue, cells or any other biological system that is capable of RNA transcription including in vitro transcription. Preferably, the animal is a non-human animal. The control is the level of the mRNA expression of ANGPTL4 in an untreated host, which may be the said host before the treatment or a different host, and/or the said host after an appropriate period of treatment for normalization to pretreatment levels. In a preferred embodiment of the method hereinbefore described, the compound that modulates PPARdelta activity is either an antagonist or an agonist.

The present invention also provides a method for monitoring treatment of patients suffering from a disease associated with dysregulation of PPARdelta activity such as for example dyslipidemia, obesity or insulin resistance, comprising the steps of:
a) purifying mRNA from muscle cells isolated from patients treated with a modulator of PPARdelta activity and
b) measuring the mRNA expression of ANGPTL4.
In one embodiment of the method hereinbefore described the mRNA expression level of ANGPTL4 is determined relative to a control.

A host may be an animal, tissue, cells or any other biological system that is capable of RNA transcription including in vitro transcription. Preferably, the animal is a non-human animal. The control is the level of the mRNA expression of ANGPTL4 in an untreated host, which may be the said host before the treatment or a different host, and/or the said host after an appropriate period of treatment for normalization to pretreatment levels.

The present invention also pertains to compounds identified by the methods hereinbefore described, and to the use of compounds identified by a method hereinbefore described for the preparation of a medicament for the treatment of a disease involving dysregulation of PPARdelta activity such as for example dyslipidemia, obesity or insulin resistance.

Several methods for measuring expression of expression levels of ANGPTL4 mRNA can be used. Methods such as Northern Blotting, and quantitation of the bands by densitometry are well known in the art and may be used, although they may not be sufficiently accurate. Other methods include the use of genechips, microarray analysis, dot blotting or different quantitative PCR methodologies. Preferably, Taqman or real time quantitative PCR is used. Any part of the transcribed sequence of ANGPTL4 may be used as probe. In a preferred embodiment, mRNA expression levels of ANGPTL4 are determined by real-time quantitative PCR using the forward primer and reverse primer listed in table 2 (SEQ. ID NOs: 4 and 5). Preferably, the mRNA expression level of ANGPTL4 in muscle cells is quantified.
The present invention further provides a method of detecting or monitoring the activity of PPARdelta in a host comprising quantifying the expression level of ANGPTL4 protein. SEQ. ID NO: 3 shows the protein sequence of mouse ANGPTL4.

In one embodiment of the method hereinbefore described the protein expression level of ANGPTL4 is determined relative to a control.

A host may be an animal, tissue, cells or any other biological system that is capable of protein translation including in vitro translation. Preferably, the animal is a non-human animal. The control is the level of the protein expression of ANGPTL4 in a different host.

Further to this the present invention provides a method of determining whether a test compound modulates PPARdelta activity in a host comprising
a) exposing the host to the test compound and
b) quantifying the protein expression level of ANGPTL4.

In one embodiment of the method hereinbefore the protein expression level of ANGPTL4 is determined relative to a control.

A host may be an animal, tissue, cells or any other biological system that is capable of protein translation including in vitro translation. Preferably, the animal is a non-human animal. The control is the level of the protein expression of ANGPTL4 in an untreated host, which may be the said host before the treatment or a different host, and/or the said host after an appropriate period of treatment for normalization to pretreatment levels. The host may be treated with a carrier. The carrier may be a solvent in which the compound is dissolved or resuspended. In a preferred embodiment of the method hereinbefore described, the compound that modulates PPARdelta activity is either an antagonist or an agonist.
Furthermore, the present invention also provides a method for monitoring treatment of patients suffering from a disease associated with dysregulation of PPARdelta activity such as for example dyslipidemia, obesity or insulin resistance, comprising the steps of:
a) purifying protein from total blood and/or muscle cells isolated from patients treated with a modulator of PPARdelta activity and
b) measuring the protein expression of ANGPTL4.
In one embodiment of the method hereinbefore the protein expression level of ANGPTL4 is determined relative to a control.

A host may be an animal, tissue, cells or any other biological system that is capable of protein translation including in vitro translation. Preferably, the animal is a non-human animal. The control is the level of the protein expression of ANGPTL4 in an untreated host, which may be the said host before the treatment or a different host, and/or the said host after an appropriate period of treatment for normalization to pretreatment levels.

The present invention also pertains to compounds identified by the methods hereinbefore described, and to the use of compounds identified by a method hereinbefore described for the preparation of a medicament for the treatment of a disease involving dysregulation of PPARdelta activity.

Several methods for measuring expression levels of ANGPTL4 protein can be used. These methods include but are not limited to two-dimensional gel separation, mass-spectrometry, antibody binding techniques (ELISA, western blot) and immunoprecipitation. Preferably, the protein expression level of ANGPTL4 in muscle cells or blood is quantified.

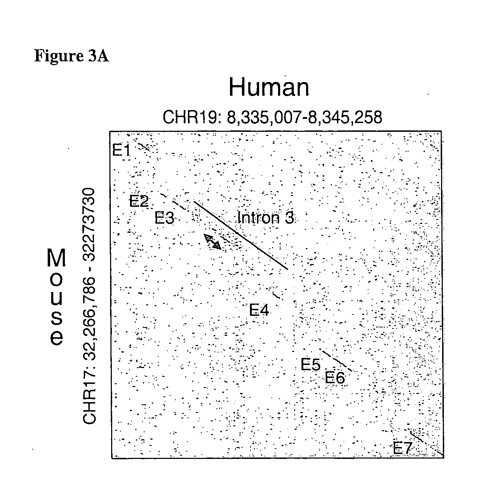

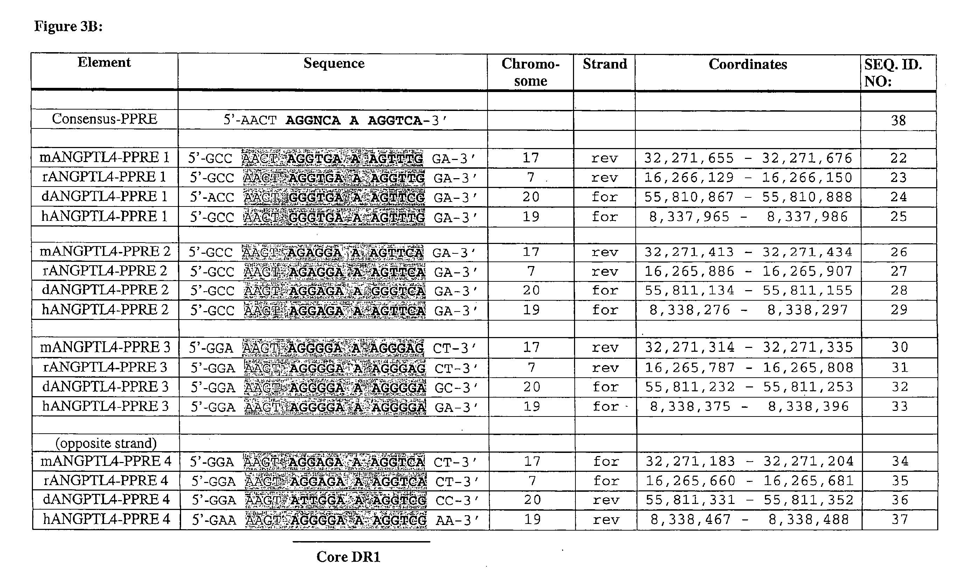

The present invention further provides a control region of FIAF/ANGPTL4 comprising binding sites for PPARs. The control region of human, mouse, rat and dog are located in the intron 3 of the corresponding ANGPTL4 gene (SEQ. ID NOs: 8-11). Preferably, the control region comprises four binding sites. The binding sites or PPAR responsive elements (PPRE's) comprise a core DR1 region of preferably 13 nucleotides (see Figure 3). The human ANGPTL4 has four PPRE's: PPRE1 (SEQ. ID NO: 25), PPRE2 (SEQ. ID NO: 29), PPRE3 (SEQ. ID NO: 33) and PPRE4 (SEQ. ID NO: 37). The mouse ANGPTL4 has four PPRE's: PPRE1 (SEQ. ID NO: 22), PPRE2 (SEQ. ID NO: 26), PPRE3 (SEQ. ID NO: 30) and PPRE4 (SEQ. ID NO: 34). The rat ANGPTL4 has four PPRE's: PPRE1 (SEQ. ID NO: 23), PPRE2 (SEQ. ID NO: 27), PPRE3 (SEQ. ID NO: 31) and PPRE4 (SEQ. ID NO: 35). The dog ANGPTL4 has four PPRE's: PPRE1 (SEQ. ID NO: 24), PPRE2 (SEQ. ID NO: 28), PPRE3 (SEQ. ID NO: 32) and PPRE4 (SEQ. ID NO: 36). The control regions and PPRE's, respectively, of other species may be identified by methods well known in the art as for example as described in Margulies E.H. and Blanchette M. (NISC Comparative Sequencing Program, Haussler, D. and Green, E.D., *Identification and Characterization of Multi-Species Conserved Sequences.* Genome Res (2003), 13:2507-2518).

The present invention further provides a regulatory sequence comprising one or more sequences or fragments thereof of the group consisting of SEQ. ID NO: 22, 26, 30, 34 (mouse), or of the group consisting of SEQ. ID NOs: 23, 27, 31, 35 (rat), or of the group consisting of SEQ. ID NOs: 24, 28, 32, 36 (dog), or of the group consisting of SEQ. ID NOs: 25, 29, 33, 37 (human). Furthermore, the present invention also provides a nucleic acid comprising SEQ. ID NO: 38 or a fragment thereof. Preferably, a fragment of a PPRE comprises the 13 core DR1 region (13 nucleotides which encompass two directly repeated (DR) similar hexanucleotide segments and one intervening nucleotide). The present invention also relates to the use of the sequences SEQ. ID NOs: 22 - 38 as regulatory sequences.

Furthermore, the present invention provides vectors comprising at least one PPRE and a host cell comprising said vector. Preferably, the vector contains the nucleic acid of SEQ. ID. NO: 16. or 17.

Host cells can be genetically engineered (i.e., transduced, transformed or transfected) to incorporate expression systems or portion thereof for polynucleotides of the present invention. Introduction of the vector into host cells can be effected by methods described in many standard laboratory manuals, such as Davis et al., Basic methods in molecular biology Elsevier, New York (1986); Davis JM (ed.): Basic cell culture: a practical approach, sec. edition. Oxford University Press (2002); R. Ian Freshney: Culture of Animal Cells: A Manual of Basic Technique, fourth edition. John Wiley & Sons (Sd) 1999; and Sambrook et al., Molecular cloning: a laboratory manual, 2. Ed., Cold Spring Harbour Laboratory Press, Cold Spring Harbor, N.Y (1989), such as calcium phosphate transfection, DEAE-dextran mediated transfection, transvectin, microinjection, cationic lipid-mediated transfection, electroporation, transduction or infection.

A host cell may be a mammalian cell such as BHK21, HEK 293, CHO, COS, HeLa, neuronal, neuroendocrinal, neuroblastomal or glial cell lines like SH-SY5Y, PC12, HN-10, bacterial cells such as streptococci, staphylococci, *E. coli, Streptomyces* and *Bacillus subtilis* cells; fungal cells such as *Saccharomyces cerevisiae* and *Aspergillus* cell; insect cells such as *Drosophila* S2 and *Spodoptera* Sf9 cells and plant cells.

A great variety of expression systems can be used. Such a system include, among others, chromosomal, episomal and virus -derived systems, i.e. vectors derived from bacterial plasmids, from bacteriophage, from transposons, from yeast episomes, from yeast chromosomal elements, from viruses such as *baculovirus,* papova viruses, such as SV40, vaccinia viruses, adenovirus, fowl pox virus, pseudorabies, retroviruses and vectors derived from combinations thereof, such as those derived from plasmids and bacteriophage genetic elements, such as cosmids and phagemids. The expression systems may contain control regions that regulate as well as engender expression. Generally, any system or vector suitable to maintain, progagate or express polynucleotides to produce a polypeptide in a host may be used. The appropriate nucleotide sequence may be inserted into an expression system by any of a variety of well-known and routine techniques, such as, for example, those set forth on Sambrook et al., Molecular cloning: a laboratory manual, 2. Ed., Cold Spring Harbour Laboratory Press, Cold Spring Harbour, N.Y. (1989) or Borowski et al Trace amines: identification of a family of mammalian G protein-coupled receptors. Proc Natl Acad Sci USA (2001) 98(16):8966-71).

The present invention further provides a method for screening of modulators of PPAR activity comprising the steps of
a) providing a host comprising one or more PPRE's operably linked to a detectable nucleic acid,
b) contacting said host with the candidate and
c) quantifying the mRNA or protein expression level of said detectable nucleic acid.
In one embodiment of the method hereinbefore described the mRNA or protein expression level of the detectable nucleic acid is determined relative to a control.

A host may be an a non-human animal, tissue, cells or any other biological system that is capable of RNA or protein transcription including in vitro transcription and in vitro translation. The control is the level of the mRNA or protein expression of the detectable nucleic acid in an untreated host, which may be the said host before the treatment, or a different host, and/or the said host after an appropriate period of treatment for normalization to pretreatment levels. In a preferred embodiment of the method hereinbefore described, the compound that modulates PPARdelta activity is either an antagonist or an agonist.

Several methods for measuring expression levels of mRNA or protein can be used. Methods for measuring expression level of mRNA include but are not limited to methods including the use of genechips, microarray analysis, dot blotting or different quantitative PCR methodologies. Preferably, Taqman or real time quantitative PCR is used. Methods for measuring expression level of protein include but are not limited to two-dimensional gel separation, mass-spectrometry, antibody binding techniques (ELISA, western blot) and immunoprecipitation.

Having now generally described this invention, the same will become better understood by reference to the specific examples, which are included herein for purpose of illustration only and are not intended to be limiting unless otherwise specified, in connection with the following figures.

### Figures

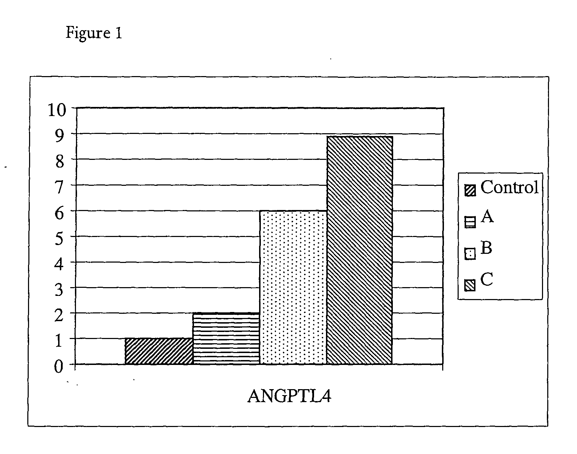

Figure 1: Graphical representation of ANGPTL4 gene expression fold changes induced by PPAR ligands in Table 1 (A 300µM, B 100nM, C 500nM) in C2C12 mouse muscle cells measured by Affymetrix micro-arrays. ANGPTL4: Angiopoietin-like proetin[marker for PPARdelta activity], A: 2-[4-(4-Chlorobenzoyl)-phenoxy]-2-methylpropanoic acid (fenofibric acid) [PPARalpha agonist], B:{2-Methyl-4-[4-Methyl-2-(4-trifluoromethyl-phenyl)-thiazol-5-ylmethylsulfanyl]-phenoxy}-acetic acid [PPARdelta-alpha co-agonist], C: [rac]-(4-{Cyclopentyl-[4-methyl-2-(4-trifluoromethylpenyl)-thiazol-5-yl]-methylsulfanyl}-naphtalen-1-yloxy}-acetic [PPAR delta agonist]

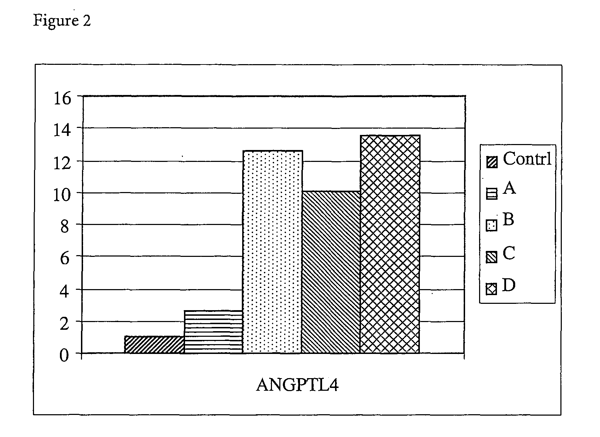

Figure 2: Graphical representation form ANGPTL4 gene expression fold changes induced by PPAR ligands in Table 3 (A 300µM, B 100nM, C 500nM, D 500nM) in C2C12 mouse muscle cells measured by qPCR. ANGPTL4: Angiopoetin-like protein 4 [marker for PPARdelta activity], A: 2-[4-(4-Chlorobenzoyl)-phenoxy]-2-methylpropanoic acid (fenofibric acid) [PPARalpha agonist], B:{2-Methyl-4-[4-Methyl-2-(4-trifluoromethylphenyl)-thiazol-5-ylmethylsulfanyl]-phenoxy}-acetic acid [PPARdelta-alpha co-agonist], C: [rac]-(4-{Cyclopentyl-[4-methyl-2-(4-trifluoromethyl-penyl)-thiazol-5-yl]-methylsulfanyl}-naphtalen-1-yloxy}-acetic [PPARdelta agonist], D: (2-Methyl-4-{Methyl-[4-methyl-2-(4-trifluoromethyl-phenyl)-thiazol-5-ylmethyl] - amino}-phenoxy)-acetic acid [PPAR delta-alpha co-agonist].
Figure 3A: Dotplot indicating nucleic acid conservation between the nucleotide sequence of the human and mouse ANGPTL 4 gene loci. E1 to 7 stands for ANGTPL4 gene exons one through seven. Intron 3 is indicated by a solid line, and the conserved ANGTPL4 control region is indicated by a double headed arrow.
Figure 3B: Alignment of the 4 peroxisome proliferator response elements (PPREs) from the human (h), mouse (m), rat (r), and dog (d) ANGPTL4 genes, showing the core DR1 elements (fat) and conserved flanking sequences compared to a consensus DR1 element. The PPAR response elements located in intron 3 of the ANGPTL4 gene identified by cross-species comparison to pinpoint conserved regulatory regions. All elements are located roughly evenly spaced in an approximately 500 nucleotide region that is highly conserved across all species. Coordinates specified refer to the following genome drafts and chromosome accession numbers: human: NCBI genome build 35.1; Jun 18, 2004 /Genbank Seq ID: NT_077812; mouse: NCBI genome build 33.1; Jun 18, 2004 / Genbank Seq ID: NT_039649; rat: NCBI; genome build 2.1; July 22, 2004 / Genbank Seq ID: NW_04773; dog: NCBI genome build 1.1; Aug 30, 2004 / Genbank Seq ID: NW_139889.

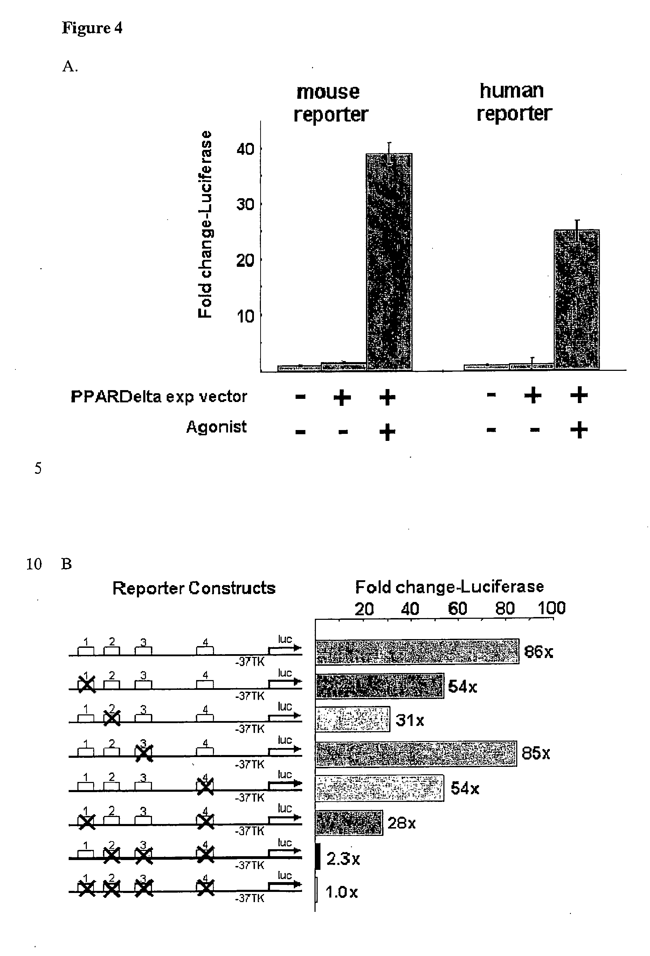

Figure 4: The ANGTPL4 control regions from mouse and human (SEQ IDs: 16, 17) were operably linked in a vector to a nucleic acid sequence encoding the firefly luciferase reporter gene (luciferase/luc). The resulting reporter vectors were transfected along with a PPARdelta expression plasmid into host cells. The transfected host was treated with 100nM PPARdelta agonist {2-Methyl-4-[4-methyl-2-(4-trifluor omethyl-phenyl)-thiazol-5-ylmethyls ulfanyl]-phenoxy}-acetic acid and luciferase activity was measured. A: Fold-induction of luciferase expression in cells transfected with a PPARdelta expression vector and either human or mouse ANGPTL4 control region luciferase reporter vectors. Luciferase is expressed as fold-activation, compared to reporter-alone expressing cells. These data indicate that both mouse and human ANGPTL4 control regions are regulated by PPARdelta. B: A similar experiment in which the PPREs in the mouse reporter construct were specifically deleted by site-directed mutagenesis. Luciferase is expressed as fold-activation by agonist in cells cotransfected with both PPARdelta and luciferase reporter. The deletion of PPREs resulted in loss of transcriptional activation mediated by PPARdelta.

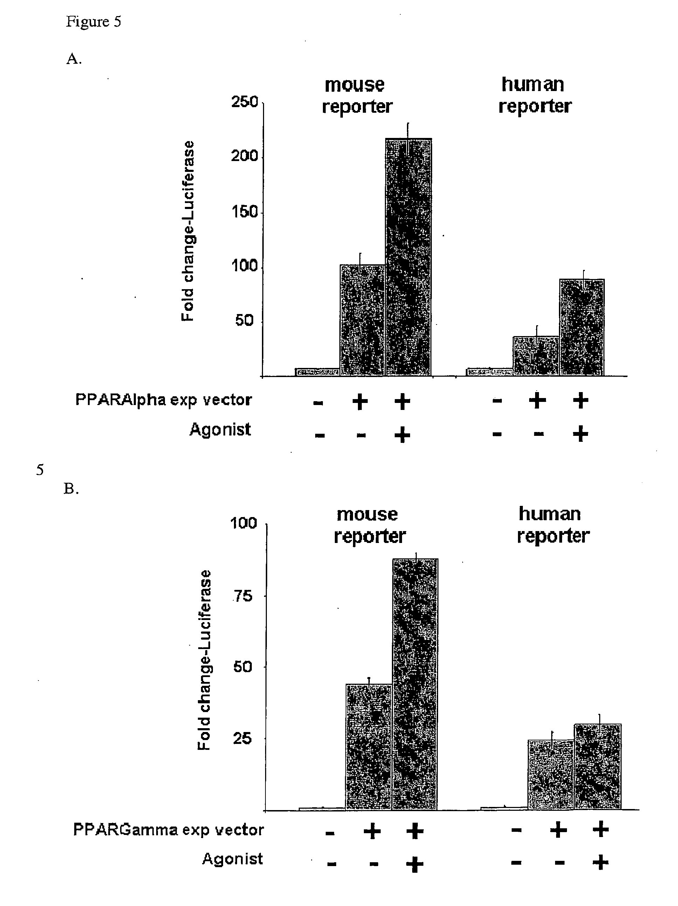

Figure 5: The ANGTPL4 control region also mediates PPARgamma and PPARalpha regulation. The mouse and human ANGTPL4 control region luciferase reporter vectors were transfected into host cells along with either a PPARalpha or PPARgamma expression vector. The transfected cells were treated with 200 µM PPARalpha agonist 2-[4-(4-Chlorobenzoyl)-phenoxy]-2-methylpropanoic acid [fenofibric acid] or 100nM PPARgamma ligand 5-[[4-[2(methyl-2-pyridinylamino)ethoxy]phenyl]- methyl]-2,4-thiazolidinedione [rosiglitazone]. Luciferase is expressed as fold-activation vs reporter-alone expressing cells.

### Examples

Commercially available reagents referred to in the examples were used according to manufacturer's instructions unless otherwise indicated.

### Example 1: Microarray experiment

### Cell culture: C2C12 cells (ATCC: CRL1772)

Cells were cultured in DMEM (5 mM glucose) supplemented with 10 % heat-inactivated fetal calf serum (FCS). Complete medium was filtered through 0.2 µm pores before use. Differentiation was performed in 6 well dishes by serum depleting the medium (DMEM High Glucose, 2% FCS) when cells reach confluency. Cell cultures were then incubated for 5 days at 37°C in a 90%-humidified and 95%-air-5%-CO₂ sterile atmosphere to allow differentiation.

PPAR Ligands were dissolved in 100% DMSO and added to medium to adjust 0.1% (v/v) DMSO final concentration and cells were incubated with ligands for 20 hours under normal growth conditions. Three replicates were done for each condition.

### RNA extraction

RNA extraction procedure has been performed with Qiagen QIAschredder and RNeasy kits.

Approximately 1*10⁶ pelleted cells per sample were resuspended in 350 µl guanidine thiocyanate containing RLT. Samples were loaded on a QIAshredder-column and centrifuged at full speed for 2 minutes to homogenize the lysates. After adding 350 µl ethanol to the flow-through for better binding conditions, samples were applied to RNeasy spin. For the first wash step 350 µl RW1 buffer were pipeted into the column. Then 10 µl DNase solution gently mixed with 70 µl RDD buffer were applied onto the column and incubated for 15 minutes at room temperature to remove genomic DNA. DNase was washed out with 350 µl RW1 buffer. Two purifying wash steps followed by adding 500 µl RPE buffer. RNA was eluted in water and stored at - 70°C.

### cDNA synthesis

cDNA was synthesized using the cDNA synthesis system kit supplied by Roche Diagnostics and following the manufacturer's manual. During the whole synthesis it was worked on ice.

For the first strand synthesis, 2 µl oligo d(T7)T₂₄ primer (5'GGCCAGTGAATTGTAATACGACTCACTATAGGGAGGCGG-(T)₂₄VN 3', 200 pmol/µl) and redist water were added to up to 20 µg RNA for a final reaction volume of 21 µl. After incubation at 70°C for 10 minutes in a thermal cycler and hybridization of the primers to the RNA, 2 µl AMV reverse transcriptase and 4 µl dNTP-mix (10 mM) were added. Supplementary 8 µl RT-buffer, 4 µl DTT (0:1 mM) and 1 µl RNase inhibitor were pipeted into the mixture. Following incubation at 42°C dured 60 minutes.

Second strand synthesis reagents were added directly into the first strand reaction tube. The second strand enzyme blend, of which 6.5 µl were applied to the mixture, contains RNase for inserting nicks into RNA of the DNA/RNA hybrid. This provides 3'OH-primers for DNA polymerase I which is also present in the 2^{nd} strand enzyme cocktail as well as E.coli ligase too. Additionally 1.5 µl dNTP-mixture, 30 µl 2^{nd} strand buffer and 72 µl redist water were added and mixed gently. The reaction mixture was incubated for 2 hours at 16°C. 20 µl added T4 polymerase ensured that the termini of the cDNA were blunt after 5 minutes incubation at 16°C. Then samples were treated with RNase to remove RNA template (30 minutes at 37°C) and proteinase K (30 minutes, 37°C).

### Purification of cDNA

Purification using the QIAquick PCR purification kit was supplied by Quiagen.

850 µl PB buffer were added to the cDNA reaction tube and mixed. Sample were applied to the QIAquick column and centrifuged for 30 seconds at 13000 rpm. 750 µl PE buffer were added to the column to wash cDNA. An additional centrifugation step at maximum speed followed which completely dried the column. To elute DNA, 50 - 80 µl EB buffer (10 mM Tris-Cl, pH: 8.5) were added to the center of the membrane and the sample was centrifuged for one minute at 13000 rpm.

The cDNA quality was checked by agarose gel electrophoresis and ethidium bromide staining.

Smears from 100 to >10000 bp appeared. The quantity and purity of the synthesized cDNA was determined by measuring the absorbance at 260 nm and 280 nm in a spectrophotometer.

The concentration was calculated as follows: c[µg/ml]= A₂₆₀* 50 *D, (50: dsDNA specific factor, D: dilution factor). cDNA has a A₂₆₀/A₂₈₀-ratio of 1.8-2.1

### In vitro Transcription (IVT)

This step was performed using MEGAscript T7 kit from Ambion.

5 µg cDNA from each samples were used as template and mixed with ATP, GTP, CTP, UTP (Ambion) and biotinylated CTP (Bio-11-CTP, ENZO) and UTP (Bio-15-UTP, ENZO). RT was performed by T7 enzyme mix for 4 hours at 37°C. Transcription products were purified with RNeasy kit from Qiagen following the same procedure as for RNA extraction, without any DNAse treatment of the samples.

### Fragmentation of IVT products

15 µg RNA from IVT were fragmented in 200 mM Tris-Acetate, pH 8.1, 500 mM KOAc, 150 mM MgOAc for 35 minutes at 95°C in a small volume (20 to 30 µl).

Chip Handling (cf DNA Micro-array Protocols V3-200.1. M Wilhelm-Seiler, U Certa)

A pre-treatment solution was first applied on Genechip (6.25 µl Acetylated BSA (20 µg/µl), 12.5 µl salmon sperm DNA (10 µg/µl), 125 µl 2x MES Hyb buffer, 106.25 µl H₂O). Genechip were incubated for 15 min at 40°C with a rotation of 60 rpm in a rotisserie.

Samples were prepared for hybridization as follows: mix 15 µg fragmented RNA with 2.5 µl control stock mix which contains BioB, BioC, BioD, Cre (internal references), 2.5 µl salmon sperm DNA (10 µg/µl), 6.25 µl Acetylated BSA (20 µg/µl), 125 µl 2x MES-Hyb buffer, 91.25 µl H₂O. Microarrays were then incubated overnight at 45°C with rotation (60 rpm) in a rotisserie.

Samples were removed and stored at -20°C. Microarrays were washed with 6 SSPE for 5 minutes in the fluidics. Then 230 µl MES-Wash buffer were applied to the chip which was incubated at 45°C for 30 minutes, 60 rpm.

Staining was performed by incubating chips with the following solution for 15 minutes: 125 µl 2x Stain Buffer , 91.25 Acetylated BSA, 2.5 µl Streptavidin (1mg/ml).

Staining solution was removed, micro-arrays washed with 6x SSPE for 5minutes in the fluidics Station, and treated with the amplification solution: 125 µl 2x Stain Buffer, 99 µl H₂O, 1 µl Biotinylated-anti-Streptavidin (500 µg/ml), 25 µl Acetylated BSA (20 µg/µl). Incubation for 30 minutes at 40°C with 60 rpm in the rotisserie. After washing I the fluidics Station with 6x SSPE, micro-arrays were incubated with phycoerytrin solution (125 µl 2x Stain buffer, 91.25 µl H₂O, 31.25 µl acetylated BSA, 2.5 µl Phycoerytrin (1 mg/ml)) at 40°C, with 60 rpm.

| **Solutions:** | |
|---|---|
| 12x MES stock | 70.4 g MES free acid monohydrate, 193.3 g MES sodium salt, adjust pH to 6.6, ad 1000 ml with tridest H₂O |
| | |
| 2x MES-Hyb Buffer | 8.3 ml 12x MES stock, 17.7 ml 5M NaCl, 4 ml 0.5M EDTA, 0.1 ml 10% Tween 20, 19.9 ml Tridest H₂O |
| | |
| MES-Wash Buffer | 83.3 ml 12x MES stock, 5.2 ml 5M NaCl, 1 ml 10% Tween 20, ad 1000 ml with tridest H₂O |
| | |
| 6x SSPE Tween | 300 ml 20x SSPE, 1 ml 10% Tween 20, ad 1000ml tridest H₂O |
| | |
| 2x Stain Buffer | 41.7 ml 12x MES stock, 92.5 ml 5M NaCl, 2.5 ml 10% Tween 20, 113.3 ml tridest H₂O |

Scanning is performed with Affymetrix scanner and results were provided on Gene Chip software from Affymetrix.

### Chip Data Analysis

Data were analyzed using Race A Sofware (Bioinformatic Roche). Triplicates were used for each condition and comparisons were calculated for each condition referring to the control (untreated cell samples). Several criteria have been taken into account for the analysis of each gene: pValue, Call average, Sum average difference, Change Factor and dispersion in each condition to get a list of the most statistically relevant genes that are modulated by the studied compounds.

### Results

A novel selective PPAR delta biomarker gene was identified from Affymetrix micro-array experiments (see Fig. 1): ANGPTL4 (angiopoetin-like protein 4) (SEQ. ID NO: 2). This marker gene allows to differentiate PPAR alpha from PPAR delta activities.

The tested ligands were a PPARalpha agonist (A: 2-[4-(4-Chlorobenzoyl)-phenoxy]-2-methylpropanoic acid (fenofibric acid)), a PPARdelta agonist (C: [rac]-(4-{Cyclopentyl-[4-methyl-2-(4-trifluoromethyl-penyl)-thiazol-5-yl] -methylsulfanyl}-naphtalen-1-yloxy}-acetic) and a PPARdelta-alpha co-agonist (B: {2-Methyl-4-[4-Methyl-2-(4-trifluoromethyl-phenyl)-thiazol-5-ylmethylsulfanyl]-phenoxy}-acetic acid).

As shown in Table 1 and Fig. 1 the response of the marker gene ANGPTL4 elicit by the PPARdelta agonist (C) and the PPARdelta-alpha co-agonist (B) is far more larger than the response elicited by the PPARalpha specific ligand (A).

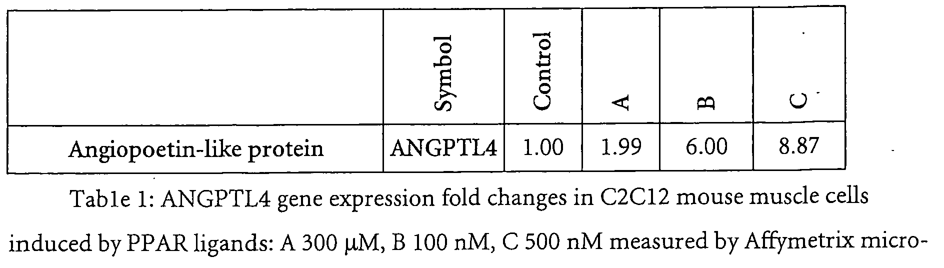

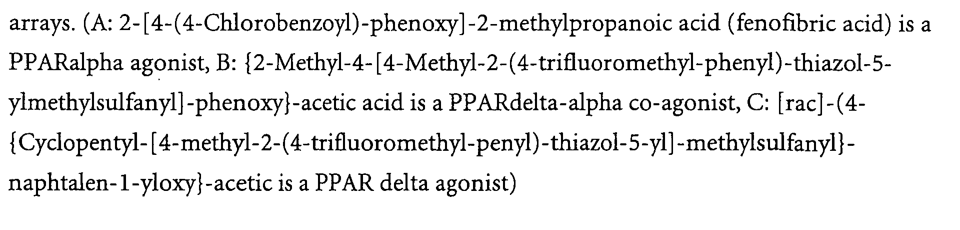

### Example 2: quantitative PCR

The culturing of the C2C12 mouse muscle cells, RNA extraction, cDNA synthesis and purifications were performed as described in Example 1.

### PCR Primer Design

Primer pairs were designed with the software Primer express 1.0 from PE Applied Biosystems based on mRNA/cDNA sequences from Medline. Criteria for each primer were length between 19 to 21 nucleotides, melting temperature of 60 °C ⁺/-1, G/C content range from 40 to 60% and avoidance of secondary structures as hairpins or primer dimers. Additionally the length of the amplicon was controlled as shorter amplicons amplify more efficiently than longer ones and are more tolerant of reaction conditions (usually ~ 100 bp). Primers were dissolved in DEPC treated water (Ambion) to a 100 µM concentrated solution and stored at -20°C.

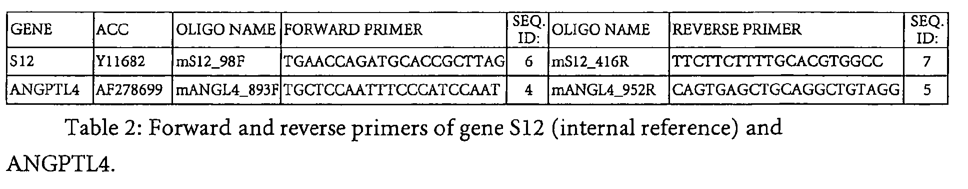

### qPCR using Corbett RG3000 and ABI 7000

RT-PCR was performed with the Quantitech SYBR Green kit from Qiagen according to the manufacturer's manual. Each component were added to 96 well plate: 25 µl master mix, 0.16 µl of each primer (100 µM stock solution), 22.7 µl H₂O and 2 µl cDNA.

The fluorescent dye SYBR green I binds the minor groove of double stranded DNA and allows measurement of DNA quantity at every cycle. The program consists of three different steps: initial denaturation of template and heat-activation of the Taq DNA polymerase (95°C, 15 min), cycling (95°C denaturation, 60 °C primer-template annealing, 72°C elongation, fluorescence aquisition), and final melting (melting curve 55 to 95°C, measurements in 0.5 °C steps). The melting curve allows the discrimination between specific and non-specific amplification products (primer dimers).

### Relative quantification using real-time PCR

The relative quantification is based on the relative expression of the target gene versus a reference gene. The concept of threshold fluorescence, defined as the point where fluorescence rises above the background fluorescence enables accurate and reproducible quantification of gene expression. The cycle number at which threshold fluorescence is reached, is called ct value. A linear relation between ct value and the log of the number of initial molecules is given during the exponential phase of the amplification reaction. Therefore, quantification is reliable at that stage and will not be affected by any limiting effects of the components. The relative ct value of a target gene compared to the ct value of an internal reference in the control or untreated sample is expressed as Δct.control = ct_{reference of control}- ct_{target of control} and in a treated, unknown sample as Δct.sample= ct_{reference of} ₛₐₘₚₗₑ- ct_{target of sample}.

The theoretical PCR reaction is expressed through the formula N = N₀* 2ⁿ (N: number of amplified molecules, N₀: initial number of molecules, n: number of amplification cycles) where the base 2 represents the optimal efficiency (one doubling every cycle). This equation allows to illustrate differential expression in mRNA copy number instead of ct values: the exponential value with the theoretical efficiency, 2 ^{Δct}, is taken.

As internal reference for quantitative studies the ribosomal protein S 12 gene expression was used. It is expressed constitutively and at the same level in all samples. Setting of fluorescence threshold is determined by the light cycler software. The obtained ct values (duplicates for one sample, two samples per condition) were exported into Microsoft Excel and analyzed as described above.

### Results

The novel selective PPAR delta biomarker gene (SEQ. ID. NO: 2) identified from Affymetrix micro-array experiments (see Fig.1) was confirmed by qPCR (quantitative polymerase chain reaction).

The tested ligands were a PPARalpha agonist (A: 2-[4-(4-Chlorobenzoyl)-phenoxy]-2-methylpropanoic acid (fenofibric acid)), a PPARdelta agonist (C:rac]-(4-{Cyclopentyl-[4-methyl-2-(4-trifluoromethyl-penyl)-thiazol-5-yl]-methylsulfanyl}-naphtalen-1-yloxy}-acetic) and two PPARdelta-alpha co-agonists (B: {2-Methyl-4-[4-Methyl-2-(4-trifluoromethyl-phenyl)-thiazol-5-ylmethylsulfanyl]-phenoxy}-acetic acid and D: (2-Methyl-4-{Methyl-[4-methyl-2-(4-trifluoromethyl-phenyl)-thiazol-5-ylmethyl]- amino}-phenoxy)-acetic acid).

As shown in Table 3 and Fig. 2 the response of the marker gene ANGPTL4 elicited by the PPARdelta agonist (C) and PPARdelta-alpha co-agonists (B and D) is far more larger than the response elicited by the PPARα specific ligand (A).

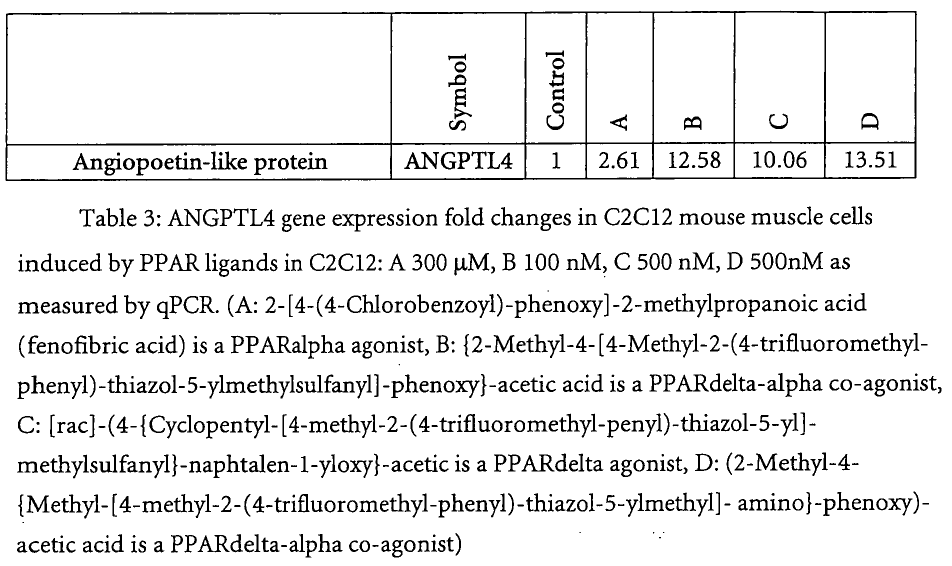

Specific PPARdelta ligands such as GW501516 are able to elicit many biological responses similar to the effects of well-known PPARalpha ligands (fibrates) both *in vitro* and *in vivo.* One major biological difference is differential regulation of ANGPTL4 expression as demonstrated by oligonucleotide arrays and quantitative PCR. ANGPTL4, a newly discovered circulating cytokine, is regulated by fasting in liver, adipocytes and muscle; it has recently been demonstrated to be a direct PPAR target gene and to be associated with lipolysis. Hence ANGPTL may provide a new approach to i. selectively distinguish the roles of PPARdelta from PPARalpha and ii. to develop more differentiated medicines for dyslipidemia and type 2 diabetes.

### Example 3:

### Comparative genomic analysis

The human (NCBI genome build 35.1; Jun 18, 2004), rat (NCBI; genome build 2.1; July 22, 2004), mouse (NCBI genome build 33.1; Jun 18, 2004) and dog (NCBI genome build 1.1; Aug 30, 2004) ANGPTL4 genes were compared. First, BLAST comparisons were perfomed to provide convenient "anchor points" for more sensitive in depth analysis of orthologous genomic regions. Then, conserved genomic regions were further analyzed using homology-based gene predictions. For the ANGPTL4 gene, various conserved stretches within the gene's introns were found as illustrated by a dot plot (Figure 3A) comparing the genomic regions of man and mouse. The best-conserved stretch is located within intron 3. It was readily detected by a BLAST search and it is even more highly conserved than the protein encoding exons of the ANGPTL4 gene.

To further elucidate the possible relevance of these conserved intronic stretches, known transcription factor binding sites (TFBSs) were mapped. All TFBSs from TransFac8.1 [BIOBASE (biological databases), Halchtersche Straße 33, D-38304 Wolfenbüttel, Germany] for which a matrix-based description was available were included. While the mapping algorithm used adjusts for statistical significance of TFBS matches, about five TFBS predictions are still obtained for any arbitrary genomic sequence stretch of 100 bp. However, based on the assumption that both regulatory mechanisms and consensus TFBSs tend to be conserved among the higher vertebrates, one can combine the TFBS predictions with alignments of the corresponding orthologous genomic regions, and with conservation profiles derived from those alignments. This allows those TFBS predictions that do not appear to be sufficiently well conserved to be excluded.

### Results

In the conserved stretch of the ANGPTL4 gene's intron 3, about 50 matches to TFBSs in TransFac8.1, including several matches to peroxisome proliferator activated receptor response element motifs (PPRE's/DR1) were identified. All four of the PPRE-related matches coincided with the four highest peaks in the conservation plot. None of the other predicted TFBSs overlapped with any of the conservation peaks. Moreover, there were no additional PPREs predicted outside of the most conserved regions. At the nucleotide level, all of the putative PPREs are well-conserved among the four species, and are excellent matches to the consensus PPRE/DR1 element (Figure 3B). Thus, the computational analyses identified four separate PPREs in intron 3 of ANGPTL4, of which three are on the forward (coding) strand and one on the reverse strand.

### Example 4:

### PCR amplification of mouse and human ANGPTL4 control region

PCR primer pairs were designed to amplify a segment of ANGPTL4 gene intron 3 from mouse and human genomic DNA encompassing the 4 putative PPREs. Primers were designed with the software Primer Express 2.0 from PE Applied Biosystems (Headquater: 850 Lincoln Centre Drive, Foster City, CA 94404, USA) using similar parameters as described in Example 2. PCR amplification was performed using the pfu PCR mix from Stratagene. Each reaction used 50 ng of template DNA, 10 pMoles each of both forward and reverse primers, with recommended amount of dNTPs, 10x buffer, and PCR enzyme in a final reaction volume of 50 µl.

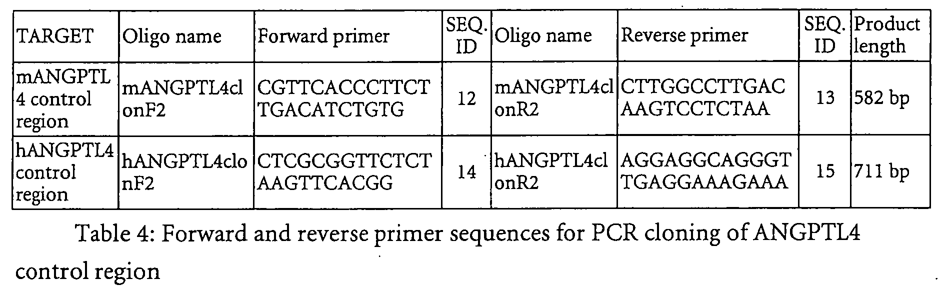

PCR amplification was carried out in a T3 thermocycler from Biometra (Whatman Biometra, Biometra GmbH i. L., Rudolf-Wissell-Straße 30, 37079 Goettingen, Germany). The amplification program consisted of initial denaturation of template (95°, 2 min), 35 repetitions at 95°, 60 sec denaturation; 50-65° Gradient, 30 sec annealing; 72°, 4 min extension. Following PCR, an aliquot was separated by agarose gel electrophoresis and quantity and size of amplified bands was visualized by ethidium bromide staining.

### Construction of ANGPTL4 control region-luciferase reporter vectors

The PCR products were subcloned into a TA vector and their DNA sequences were determined. The inserted PCR products with the expected sequence were recovered by restriction digestion of the insert-containing TA plasmids and subcloned into a luciferase reporter vector containing a minimal -37 TK promoter (pGL3basic-pTK-37Luc: insertion of minimal promoter region from the herpes simplex thymidine kinase gene (pTK37) (SEQ. ID NO: 39) between BgIII and EcoRI restriction sites of the pGL3-Basic vector (Promega, Aceession number: U47295)). This resulted in two plasmids; mANGPTL4controlregion-Luc and hANGPTL4controlregion-Luc in which luciferase expression is directed by the ANGPTL4 control region from mouse and human, respectively. Large amounts of these plasmids were prepared in *E coli* for subsequent studies.

### Mutagenesis

To verify that PPARdelta transcriptional control of the ANGPTL4 control region is dependent upon the 4 identified PPREs, they were made nonfunctional individually and in combination, by site-directed mutagenesis of the mANGPTL4controlregion-Luc plasmid. Primers were produced to delete the 13 core DR1-encoding nucleotides of each PPRE. Mutagenesis was carried out using the Quikchange Multi Site Directed Mutagenesis Kit from Stratagene. Properly mutagenized vectors containing deletion(s) of one or more of the PPREs were identified by DNA sequencing. Large amounts of these plasmids were prepared in *E coli* for subsequent studies.

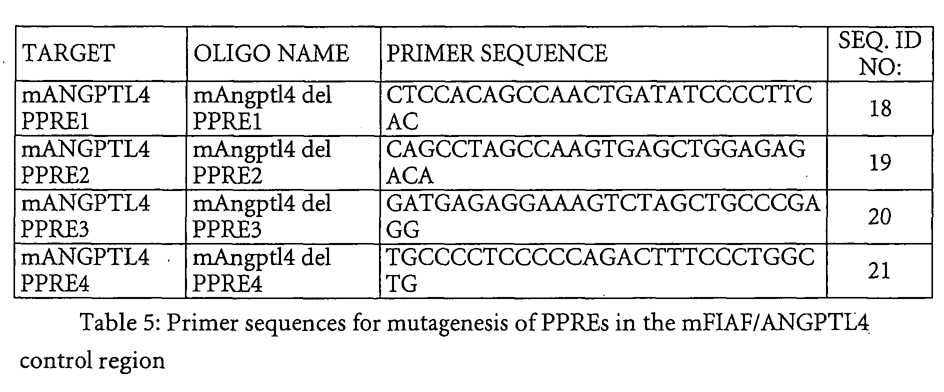

### Luciferase assays

Baby hamster kidney cells (BHK21 ATCC CCL10) were grown in DMEM medium containing 10% FBS at 37°C in a 95%O2:5%CO₂ atmosphere. Cells were seeded in 6 well plates at a density of 2 x 10⁵ cells/well and then batch-transfected with vectors expressing full-length human PPARδ, PPARγ or PPARα receptors along with the appropriate ANGPTL4controlregion-Luc reporter plasmid. Transfection was accomplished with the Fugene 6 reagent (Roche Molecular Biochemicals) according to the suggested protocol. Six hours following transfection, the cells were harvested by trypsinization and seeded in 96 well plates at a density of 10⁴ cells/well. After 24 hours to allow attachment of cells, the medium was removed and replaced with 100 µl of phenol red-free DMEM medium containing the test substances or control ligands (final DMSO concentration: 0.2%). Following incubation of the cells for 24 hours with substances, 50 µl of the supernatant was discarded and then 50 µl of Steady-Glo Luciferase Assay Reagent (Promega) to lyse the cells and initiate the luciferase reaction was added. Luminescence for luciferase was measured in a Packard TopCount. Transcriptional activation in the presence of a test substance was expressed as fold-activation over cells incubated in the absence of the substance.

### Results

Figure 4A shows that PPARdelta mediates transcriptional control of the heterologous luciferase reporter via the FIAF/ANGPTL4 control regions. Cells transfected with PPARdelta receptor vector along with luciferase reporter showed no increase in transcriptional activation compared to cells transfected with the reporter alone. However, after treatment with PPARdelta agonist, luciferase activity increased by 39-fold and 25-fold for the mouse and human FIAF/ANGPTL4 control region-Luc reporters, respectively. In Figure 4B, a similar experiment was performed but using mFIAF/ANGPTL4 control region-Luc reporters in which one or more of the PPREs was deleted. Deletion of PPREs 1, 2, and 4 alone caused a decrease in PPARdelta-mediated transcription. Deletion or more than one PPRE resulted in further losses in transcriptional activation with all regulation lost upon deletion of all four PPREs. Thus PPARdelta robustly and specifically regulates transcription via the 4 PPREs contained within the mouse and human FIAF/ANGPTL4 control regions.

Since PPARalpha and PPARgamma agonists have been shown to induce FIAF/ANGPTL4 expression in liver and adipose tissue, respectively, they were also tested for regulation of the ANGPTL4 control region. Figure 5 shows that both PPARalpha and PPARgamma can transcriptionally regulate the mouse and human ANGPTL4 control regions. In contrast to PPARdelta, transfection of cells with PPARalpha and PPARgamma resulted in increased activation even in the absence of agonist, that was further increased upon treatment with selective agonists.

## Claims

1. ANGPTL4 as biomarker for determining PPARdelta activity.

2. ANGPTL4 as biomarker for determining PPARdelta activity in muscle cells or liver or adipocytes.

3. ANGPTL4 as a marker for diagnosing a disease involving dysregulation of PPARdelta activity.

4. A method of detecting or monitoring the activity of PPARdelta in a host comprising quantifying the mRNA expression level of ANGPTL4.

5. A method according to claim 4 comprising the step of determining the mRNA expression level of ANGPTL4 relative to a control.

6. A method of determining whether a test compound modulates PPARdelta activity in a host comprising
a) exposing the host to the test compound and
b) quantifying the mRNA expression level of ANGPTL4.

7. A method according to claim 6 comprising determining the mRNA expression level of ANGPTL4 relative to a control.

8. A method for monitoring treatment of patients suffering from a disease associated with dysregulation of PPARdelta activity, comprising the steps of
a) purifying RNA from muscle cells isolated from patients treated with a modulator of PPARdelta activity and
b) measuring the mRNA expression of ANGPTL4.

9. A method according to claim 8 comprising determining the mRNA expression level of ANGPTL4 relative to a control.

10. Compounds identified by the methods of any one of the claims 4 to 9.

11. Use of compounds identified by a method of any one of the claims 4 to 9 for the preparation of a medicament for the treatment of a disease involving dysregulation of PPARdelta activity.

12. A method of detecting or monitoring the activity of PPARdelta in a host comprising quantifying the protein expression level of ANGPTL4.

13. A method according to claim 12 comprising the step of determining the protein expression level of ANGPTL4 relative to a control.

14. A method of determining whether a test compound modulates PPARdelta activity in a host comprising
a) exposing the host to the test compound and
b) quantifying the protein expression level of ANGPTL4.

15. A method according to claim 14 comprising determining the protein expression level of ANGPTL4 relative to a control.

16. A method for monitoring treatment of patients suffering from a disease associated with dysregulation of PPARdelta activity, comprising the steps of
a) purifying protein from total blood and/or muscle cells isolated from patients treated with a modulator of PPARdelta activity and
b) measuring the protein expression of ANGPTL4.

17. A method according to claim 16 comprising determining the protein expression level of ANGPTL4 relative to a control.

18. Compounds identified by a method according to any one of the claims 12 to 17.

19. Use of compounds identified by a method according to any one of the claims 12 to 17 for the preparation of a medicament for the treatment of a disease involving dysregulation of PPARdelta activity.

20. Use of ANGPTL4 as biomarker for determining PPARdelta activity.

21. Use of ANGPTL4 as biomarker for determining PPARdelta activity in muscle.

22. Use of ANGPTL4 as a marker for diagnosing a disease involving dysregulation of PPARdelta activity.

23. A regulatory sequence comprising one or more sequences or fragments thereof of the group consisting of SEQ. ID NO: 22, 26, 30, 34,

24. A regulatory sequence comprising one or more sequences or fragments thereof of the group consisting of SEQ. ID NOs: 23 27, 31, 35.

25. A regulatory sequence comprising one or more sequences or fragments thereof of the group consisting of SEQ. ID NOs: 24, 28, 32, 36.

26. A regulatory sequence comprising one or more sequences or fragments thereof of the group consisting of SEQ. ID NOs: 25, 29, 33, 37.

27. A regulatory sequence comprising SEQ. ID NO: 38 or a fragment thereof.

28. A method for screening of modulators of PPAR activity comprising the steps of
a) providing a host comprising one or more PREE's operably linked to a detectable nucleic acid,
b) contacting said host with the candidate and
c) quantifying the mRNA or protein expression level of said detectable nucleic acid.

29. A method according to claim 28 comprising determining the mRNA or protein expression level of the detectable nucleic acid relative to a control.
